(19) Europäisches Patentamt
European Patent Office
Office européen des brevets

(11) EP 1 562 979 B1

(12) **EUROPÄISCHE PATENTSCHRIFT**

(45) Veröffentlichungstag und Bekanntmachung des Hinweises auf die Patenterteilung:
**29.10.2008 Patentblatt 2008/44**

(21) Anmeldenummer: **03775204.5**

(22) Anmeldetag: **20.10.2003**

(51) Int Cl.:
*C07K 5/02* (2006.01) *C07D 207/26* (2006.01)
*C07D 277/56* (2006.01) *C07D 417/12* (2006.01)
*C07F 7/18* (2006.01) *C07C 271/22* (2006.01)
*C07D 211/60* (2006.01) *A61K 38/08* (2006.01)

(86) Internationale Anmeldenummer:
**PCT/EP2003/011603**

(87) Internationale Veröffentlichungsnummer:
**WO 2004/046170 (03.06.2004 Gazette 2004/23)**

(54) **TUBULYSINE, HERSTELLUNGSVERFAHREN UND TUBULYSIN-MITTEL**

TUBULYSINS, METHOD FOR PRODUCING THE SAME AND TUBULYSIN PREPARATIONS

TUBULYSINES, PROCEDE DE FABRICATION ET AGENT CONTENANT DE LA TUBULYSINE

(84) Benannte Vertragsstaaten:
**AT BE BG CH CY CZ DE DK EE ES FI FR GB GR HU IE IT LI LU MC NL PT RO SE SI SK TR**

(30) Priorität: **21.11.2002 DE 10254439**

(43) Veröffentlichungstag der Anmeldung:
**17.08.2005 Patentblatt 2005/33**

(73) Patentinhaber: **Helmholtz-Zentrum für Infektionsforschung GmbH**
**38124 Braunschweig (DE)**

(72) Erfinder:
- **HOEFLE, Gerhard**
  **38124 Braunschweig (DE)**
- **GLASER, Nicole**
  **38124 Braunschweig (DE)**
- **STEINMETZ, Heinrich**
  **38124 Braunschweig (DE)**
- **LEIBOLD, Thomas**
  **38124 Braunschweig (DE)**
- **SASSE, Florenz**
  **38124 Braunschweig (DE)**

(74) Vertreter: **Boeters, Hans Dietrich et al**
**BOETERS & LIECK**
**Oberanger 32**
**80331 München (DE)**

(56) Entgegenhaltungen:
**WO-A-20/04005327**

- **HÖFLE G ET AL.: "Semisynthesis and degradation of the tubulin inhibitors epothilone and tubulysin" PURE AND APPLIED CHEMISTRY, Bd. 75, Nr. 2-3, Februar 2003 (2003-02), Seiten 167-178, XP002379278 ISSN: 0033-4545**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Tubulysine sind als Verbindungen der folgenden allgemeinen Formel bekannt; vgl. beispielsweise F. Sasse, H. Steinmetz, J. Heil, G. Höfle, H. Reichenbach, J. Antibiot. 2000, 53, 579-558, und H. Reichenbach, G. Höfle, F. Sasse, H. Steinmetz (GBF), DE 196 38 870 A1, 1996.

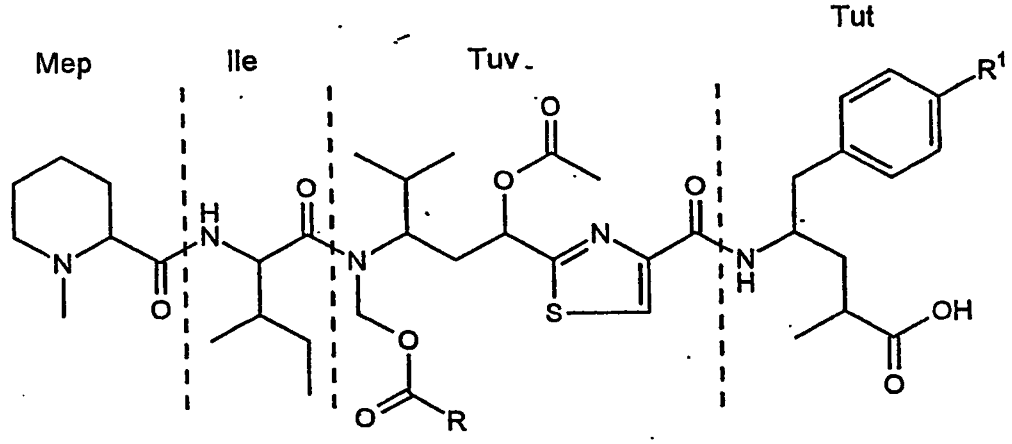

| | | R | $R^1$ |
|---|---|---|---|
| **1** | Tubulysin A | *i*-$C_4H_9$ | OH |
| **2** | Tubulysin B | $C_3H_7$ | OH |
| **3** | Tubulysin C | $C_2H_5$ | OH |
| **4** | Tubulysin D | *i*-$C_4H_9$ | H |
| **5** | Tubulysin E | $C_3H_7$ | H |
| **6** | Tubulysin F | $C_2H_5$ | H |

Aufgabe der Erfindung ist es, neue Tubulysine, Verfahren zu ihrer Herstellung und Mittel mit Tubulysinen bereitzustellen, insbesondere als Cytostatika.

Die Erfindung betrifft eine Verbindung der folgenden allgemeinen Formel I (Tubulysin):

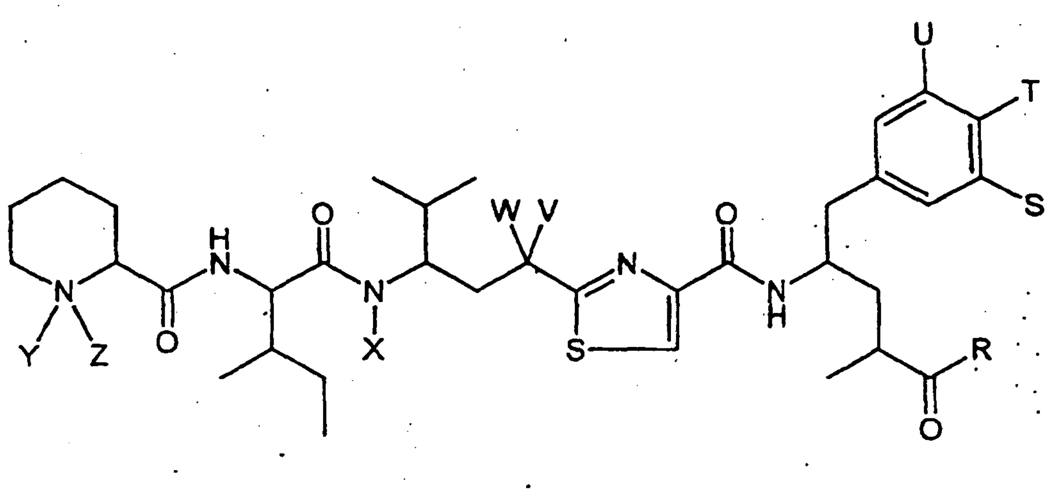

mit den folgenden Bedeutungen für R, $R^1$, $R^4$, $R^5$, $R^6$, $R^7$, $R^8$, $R^9$, $R^{10}$, $R^{11}$, S, T, U, V, W, X, Y und Z:

$R = OR^1$
$R^1$ = Alkyl oder Aryl
S = H
U = H
T = H oder $OR^4$
$R^4$ = H, Alkyl, Aryl, $COR^5$, $P(O)(OR^6)_2$ oder $SO_3R^6$
$R^5$= Alkyl, Alkenyl, Aryl oder Heteroaryl
$R^6$ = H, Alkyl oder Metallion
$V = OR^7$
$R^7 = COR^8$
$R^8$ = Alkyl, Alkenyl oder Aryl
W = H
$X = CH_2OR^9$
$R^9 = COR^{10}$
$R^{10}$ = Alkyl, Alkenyl, Aryl oder Heteroaryl
Y = freies Elektronenpaar
$R^{11}$ = Alkyl, $CF_3$ oder Aryl und/oder
$Z = CH_3$ oder $COR^{11}$.

Bei Alkyl kann es sich um verzweigtes, unverzweigtes oder zyklisches $C_{1-20}$-Alkyl, insbesondere $C_{1-7}$-Alkyl, vorzugsweise $C_{1-6}$-Alkyl und besonders bevorzugt um $C_{1-4}$-Alkyl, insbesondere Methyl, Ethyl, Propyl, Isopropyl, n-Butyl, iso-Butyl, sek-Butyl, tert-Butyl, handeln. Cycloalkyl hat vorzugsweise 3 bis 8 C-Atome im Ring

Bei den Alkenylgruppen kann es sich um verzweigtes, unverzweigtes oder zyklisches $C_{2-20}$-Alkenyl, insbesondere $C_{2-7}$-Alkenyl, vorzugsweise $C_{2-6}$-Alkenyl und besonders bevorzugt um $C_{2-4}$-Alkenyl, insbesondere Vinyl, Allyl, Propen-1-yl, Propen-2-yl, But-1-en-1-yl, But-1-en-2-yl, But-1-en-3-yl, But-1-en-4-yl, But-2-en-1-yl, But-2-en-2-yl, 2-Methyl-propen-1-yl, 2-Methyl-propen-3-yl, handeln. Cycloalkenyl hat vorzugsweise 3 bis 8 C-Atome im Ring. Die Anzahl der Doppelbindungen der Alkenylgruppen kann 1 bis 3 betragen.

Aryl kann sein Phenyl, Naphthyl und Biphenylyl.

Heteroaryl kann sein Furyl, Thienyl, Imidazolyl, Indolyl, Pyridinyl, Pyrrolyl, Thiazolyl, Oxazolyl und Pyrimidinyl.

Alkyl, Alkenyl, Aryl und Heteroaryl können unsubstituiert oder substituiert sein, so können sie in beliebiger Position 1 bis 3 Substituenten aus der durch $C_{1-3}$-Alkyl, $C_{1-3}$-Alkoxy, Hydroxy, Amino ($NE_2$) oder Nitro ($NO_2$) gebildeten Gruppe tragen.

So kann eine erfindungsgemäße Verbindung aufweisen:

R, $R^1$, $R^4$, $R^5$, $R^8$, $R^9$, $R^{10}$ und/oder $R^{11}$ = unsubstituiertes oder substituiertes Phenyl, insbesondere $C_{1-4}$-Alkyl-substituiertes Phenyl
$R^5$ = $C_{1-4}$-Alkyl, $C_{2-6}$-Alkenyl oder Pyridyl
$R^5$ und/oder X = $C_{2-4}$-Alkenyl
$R^6$ = Alkalimetall-Ion, insbesondere Na-Ion, oder Erdalkalimetall-Ion
$R^8$ und/oder $R^9$ = $C_{2-4}$-Alkenyl und/oder
$R^{10}$ = $C_{2-6}$-Alkenyl, insbesondere $C_{2-4}$-Alkenyl, oder Pyridyl.

Schema 1 zeigt ein Verfahren zur Herstellung einer Verbindung der allgemeinen Formel I (Typ 7) mit $R = OR^1$, $R^1$ = H, S = U = H, T = H oder OH, $V = OR^7$, $R^7 = COR^8$, $R^8$ = Alkyl, vorzugsweise $C_{1-4}$-Alkyl, insbesondere Methyl, W = H, $X = CH_2OR^9$, $R^9$ = H, Y = freies Elekronenpaar und $Z = CH_3$, bei dem man eine Verbindung der folgenden allgemeinen Formel II (Typ 1, 2, 3, 4, 5 oder 6) :

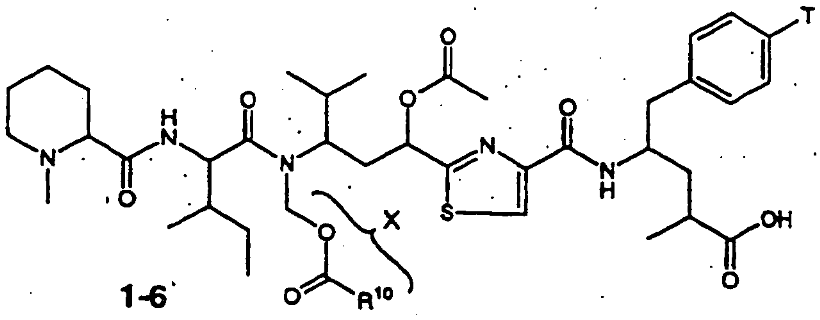

1-6

mit X = $CH_2OR^9$, $R^9$ = $COR^{10}$, $R^{10}$ = Alkyl und insbesondere $C_{1-6}$-Alkyl und im übrigen den vorstehend angegebenen Bedeutungen einer Esterspaltung in saurem Medium unterwirft und die Verbindung der allgemeinen Formel I mit den angegebenen Bedeutungen gewinnt.

Bei dem Verfahren kann man die Esterspaltung in einem organischen Lösungsmittel, insbesondere Dioxan, in Gegenwart einer Säure, insbesondere Chlorwassertoff, und/oder bei erhöhter Temperatur durchführen.

Schema 1 zeigt ferner ein Verfahren zur Herstellung einer Verbindung der allgemeinen Formel I (Typ 8) mit R = $OR^1$, $R^1$ = H, S = U = H, T = H oder OH, V = $OR^7$, $R^7$ = $COR^8$, $R^8$ = Alkyl und insbesondere $C_{1-4}$-Alkyl, insbesondere Methyl, W = H, X = H, Y = freies Elektronenpaar und Z = $CH_3$, bei dem man eine Verbindung der allgemeinen Formel II (Typ 1, 2, 3, 4, 5 oder 6) mit X = $CH_2OR^9$, $X^9$ = $COR^{10}$, $R^{10}$ = Alkyl und insbesondere $C_{1-6}$-Alkyl, und im übrigen den vorstehend angegebenen Bedeutungen einer Acetal-Spaltung unterwirft und die Verbindung der allgemeinen Formel I mit den angegebenen Bedeutungen gewinnt.

Bei dem Verfahren kann man die Acetal-Spaltung in saurem Milieu, insbesondere in Gegenwart von Salzsäure, und/oder bei erhöhter Temperatur durchführen.

Schema 1 zeigt ferner ein Verfahren zur Herstellung einer Verbindung der allgemeinen Formel I (Typ 9) mit R = $OR^1$, $R^1$ = H, S = U = H, T = H oder OH, V = $OR^7$, $R^7$ = H, W = H, X = $CH_2OR^9$, $R^9$ = $COR^{10}$, $R^{10}$ = Alkyl und insbesondere $C_{1-6}$-Alkyl, Y = freies Elektronenpaar und Z = $CH_3$, bei dem man eine Verbindung der allgemeinen Formel II (Typ 1, 2, 3, 4, 5 oder 6) mit V = $OR^7$, $R^7$ = $COR^8$, $R^8$ = Alkyl, vorzugsweise $C_{1-4}$-Alkyl, insbesondere Methyl, und im übrigen den vorstehend angegebenen Bedeutungen einer Esterspaltung in schwach alkalischem Medium unterwirft und die Verbindung der allgemeinen Formel I mit den angegebenen Bedeutungen gewinnt.

Bei dem Verfahren kann man die Esterspaltung in einem organischen Medium, insbesondere einem hydrophilen organischen Lösungsmittel, vorzugsweise einem Alkohol, insbesondere Methanol, in Gegenwart einer schwachen Base durchführen, insbesondere $NH_3$.

Schema 1 zeigt ferner ein Verfahren zur Herstellung einer Verbindung der allgemeinen Formel I (Typ 10) mit R = $OR^1$, $R^1$ = H, S = U = H, T = H oder OH, V = $OR^7$, $R^7$ = H, W = H, X = H, Y = freies Elektronenpaar und Z = $CH_3$, bei dem man eine Verbindung der allgemeinen Formel II (Typ 1, 2, 3, 4, 5 oder 6) mit V = $OR^7$, $R^7$ = $COR^8$, $R^8$ = Alkyl , vorzugsweise $C_{1-4}$-Alkyl, insbesondere Methyl, X = $CH_2OR^9$, $R^9$ = $COR^{10}$, $R^{10}$ = Alkyl und insbesondere $C_{1-6}$-Alkyl, und im übrigen den vorstehend angegebenen Bedeutungen einer doppelten Esterspaltung in stark alkalischem Medium unterwirft und die Verbindung der allgemeinen Formel I mit den angegebenen Bedeutungen gewinnt.

Bei dem Verfahren kann man die doppelte Esterspaltung in einem organischen Medium, insbesondere in einem hydrophilen organischen Lösungsmittel, vorzugsweise Alkohol, insbesondere Methanol, in Gegenwart einer starken Base durchführen, insbesondere eines Alkalimetallhydroxids, vorzugsweise von Natriumhydroxid.

Schema 1 zeigt ferner ein Verfahren zur Herstellung einer Verbindung der folgenden allgemeinen Formel III (Typ 11) :

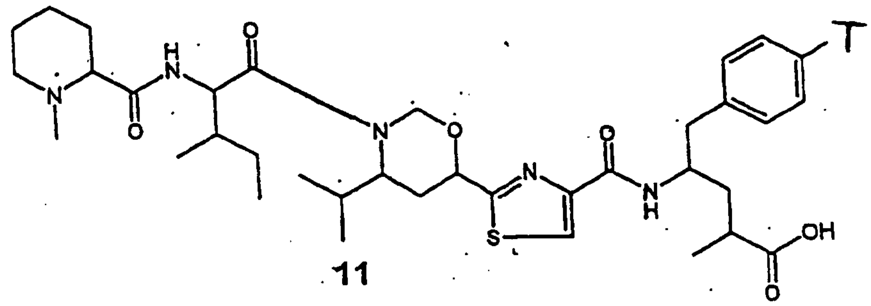

11

mit R = $OR^1$, $R^1$ = H, S = U = H, T = H oder $OR^4$, $R^4$ = H, V mit X = $CH_2O$-Brücke, W = H, Y = freies Elektronenpaar und Z = $CH_3$ in der allgemeinen Formel I, bei dem man eine Verbindung der allgemeinen Formel II (Typ 1, 2, 3, 4, 5 oder 6) mit X = $CH_2OR^9$, $R^9$ = $COR^{10}$, $R^{10}$ = Alkyl und insbesondere $C_{1-6}$-Alkyl, V = $OR^7$, $R^7$ = $COR^8$, $R^8$ = Alkyl, vorzugsweise $C_{1-4}$-Alkyl, insbesondere Methyl, und im übrigen den vorstehend angegebenen Bedeutungen einer Ringbildung unter doppelter Esterspaltung in saurem Medium unterwirft und die Verbindung der vorstehenden allgemeinen Formel mit den angegebenen Bedeutungen gewinnt.

Bei dem Verfahren kann man die Ringbildung in wässerigem Medium, in Gegenwart einer anorganischen Säure, vorzugsweise Salzsäure, und unter Erhitzen durchführen.

Schema 2 zeigt ein Verfahren zur Herstellung einer Verbindung der allgemeinen Formel I (Typ 12) mit R = $OR^1$, $R^1$ = H, S = U = H, T = H oder $OR^4$, $R^4$ = $COR^5$, $R^5$ = Alkyl und insbesondere $C_{1-6}$-Alkyl, Alkenyl und insbesondere $C_{2-6}$-Alkenyl, Aryl oder Heteroaryl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = $R^5$, Y = freies Elektronenpaar und Z = $CH_3$, bei dem man eine Verbindung der folgenden allgemeinen Formel IV (Typ 7):

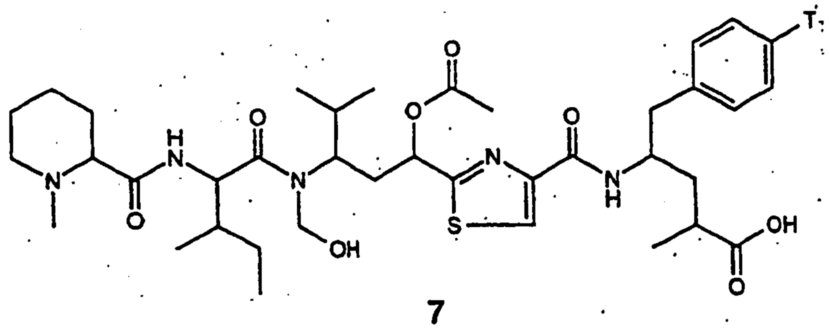

7

mit X = $CH_2OR^9$, $R^9$ = H und im übrigen den vorstehend angegebenen Bedeutungen einer Acylierung unterwirft und eine Verbindung der allgemeinen Formel I mit den angegebenen Bedeutungen gewinnt.

Bei dem Verfahren kann man mit einem Acylhalogenid, insbesondere Acylchlorid, und/oder in Gegenwart einer schwachen Base acylieren, insbesondere einer schwachen organischen Base, vorzugsweise eines tertiären Amins, insbesondere Triethylamin.

Schema 2 zeigt ferner ein Verfahren zur Herstellung einer Verbindung der allgemeinen Formel I (Typ 13) mit R = $OR^1$, $R^1$ = H, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man bei einem Produkt mit T = $OR^4$, $R^4$ = $COR^5$ und $R^5$ = Alkyl und insbesondere $C_{1-6}$-Alkyl, Alkenyl und insbesondere $C_{2-6}$-Alkenyl, Aryl oder Heteroaryl und im übrigen den vorstehend angegebenen Bedeutungen in alkalischem Medium verseift und eine Verbindung der allgemeinen Formel I mit den angegebenen Bedeutungen gewinnt.

Bei dem Verfahren kann man mit Ammoniak verseifen.

Schema 3 zeigt ein Verfahren zur Herstellung einer Verbindung der allgemeinen Formel I (Typ 14) mit R = $DR^1$, $R^1$ = H, S = U = H, T = H oder OH, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = Alkyl und insbesondere $C_{1-4}$-Alkyl, Alkenyl und insbesondere $C_{2-6}$-Alkenyl, oder Aryl, Y = freies Elektronenpaar und Z = $CH_3$, bei dem man Verbindung der allgemeinen Formel II (Typ 1, 2, 3, 4, 5 oder 6) einer Esterspaltung unterwirft und alkyliert und eine Verbindung der allgemeinen Formel I mit den angegebenen Bedeutungen gewinnt.

Bei dem Verfahren kann man mit einem Alkylierungsmittel der Formel $R^9OH$ mit $R^9$ = Alkyl und insbesondere $C_{1-4}$-Alkyl, Alkenyl oder Aryl umsetzen.

Bei dem Verfahren kann man in Gegenwart von p-$CH_3$-$C_6H_4SO_2OH$ in Tetrahydrofuran (THF) bei erhöhter Temperatur umsetzen.

Schema 4 zeigt ein Verfahren zur Herstellung einer Verbindung der allgemeinen Formel I (Typ 15) mit R = $OR^1$, $R^1$ = H, S = U = H, T = H oder $OR^4$, $R^4$ = H, V = $OR^7$, $R^7$ = H oder $COR^8$, $R^8$ = Alkyl, vorzugsweise $C_{1-4}$-Alkyl, insbesondere Methyl, W = H, X = $CH_3$, Y = freies Elektronenpaar und Z = $CH_3$, bei dem man eine Verbindung der allgemeinen Formel IV (Typ 7) mit X = $CH_2OR^9$, $R^9$ = H und im übrigen den vorstehend angegebenen Bedeutungen einer Reduktion unterwirft und die Verbindung der allgemeinen Formel I mit den angegebenen Bedeutungen gewinnt.

Bei dem Verfahren kann man die Reduktion mit $NaCNBH_3$ und Trifluoressigsäure in Methanol (MeOH) durchführen.

Schema 4 zeigt ferner ein Verfahren zur Herstellung einer Verbindung der allgemeinen Formel I (Typ 15) mit R = $OR^1$, $R^1$ = H, S = U = H, T = H oder $OR^4$, $R^4$ = H, V = $OR^7$, $R^7$ = H oder $COR^8$, $R^8$ = Alkyl, vorzugsweise $C_{1-4}$-Alkyl, insbesondere Methyl, W = H, X = $CH_3$, Y = freies Elektronenpaar und Z = $CH_3$, bei dem man eine Verbindung der allgemeinen Formel III (Typ 11) einer Ringöffnung unter Reduktion bzw. Reduktion unter Ringöffnung unterwirft und eine Verbindung der allgemeinen Formel I mit den angegebenen Bedeutungen gewinnt.

Bei dem Verfahren kann man in Gegenwart von $NaCNBH_3$ und $Me_3SiCl$ in Acetonitril ($CH_3CN$) umsetzen.

Schema 5 zeigt ein Verfahren zur Herstellung einer Verbindung der allgemeinen Formel I (Typ 16) mit R = $OR^1$, $R^1$ = H, S = U = H, T = H oder OH, V = $OR^7$, $R^7$ = $COR^8$, $R^8$ = Alkyl und insbesondere $C_{1-4}$-Alkyl, Alkenyl oder Aryl, W = H, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man eine Verbindung der allgemeinen Formel I (Typ 9) mit V = $OR^7$ und $R^7$ = H und im übrigen den vorstehend angegebenen Bedeutungen einer Acylierung unterwirft und die Verbindung der allgemeinen Formel I mit den angegebenen Bedeutungen gewinnt.

Bei dem Verfahren kann man die Acylierung mit einem Acylhalogenid der Formel $R^8COCl$ mit $R^8$ = Alkyl und insbesondere $C_{1-4}$-Alkyl, Alkenyl oder Aryl, insbesondere Acylchlorid, und/oder in Gegenwart einer Base durchführen, insbesondere einer organischen Base, vorzugsweise eines Trialkylamins, insbesondere Triethylamin.

Schema 5 zeigt ferner ein Verfahren zur Herstellung einer Verbindung der allgemeinen Formel I (Typ 17) mit R = $OR^1$, $R^1$ = H, S = U = H, T = H oder $OR^4$, $R^4$ = H, V = H oder F, W = H, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man eine Verbindung der allgemeinen Formel I (Typ 9) mit V = $OR^7$ und $R^7$ = H und im übrigen den vorstehend angegebenen Bedeutungen einer katalytischen Hydrierung oder einer Fluorierung unterwirft und die Verbindung der allgemeinen Formel I mit den angegebenen Bedeutungen gewinnt.

Bei dem Verfahren kann man für V = H die Hydrierung mit Palladium/Kohlenstoff in Gegenwart von Essigsäure oder für V = F die Fluorierung mit DAST in Tetrahydrofuran durchführen.

Schema 5 zeigt ferner ein Verfahren zur Herstellung einer Verbindung der allgemeinen Formel I (Typ 18) mit R = $OR^1$, $R^1$ = H, S = U = H, T = H oder $OR^4$, $R^4$ = H, V mit W = O, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man eine Verbindung der allgemeinen Formel I (Typ 9) mit V = $OR^7$ und $R^7$ = H und im übrigen den vorstehend angegebenen Bedeutungen einer Oxidation unter Bildung eines Ketons unterwirft und eine Verbindung der allgemeinen Formel I mit den angegebenen Bedeutungen gewinnt.

Bei dem Verfahren kann man die Oxidation in Gegenwart von TPAP und NMO in Dichlormethan durchführen.

Schema 5 zeigt ferner ein Verfahren zur Herstellung einer Verbindung der allgemeinen Formel I (Typ 19) mit R = $OR^1$, $R^1$ = H, S = U = H, T = H oder OH, V = $OR^7$, $R^7$ = H, W = Alkyl und insbesondere $C_{1-4}$-Alkyl, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man ein Produkt des vorstehenden Verfahrens (Typ 18) mit einer Grignard-Verbindung zur Verbindung der allgemeinen Formel I mit den angegebenen Bedeutungen umsetzt.

Bei dem Verfahren kann man die Umsetzung mit einer magnesiumorganischen Verbindung der Formel WMgHal mit W = Alkyl und insbesondere $C_{1-4}$-Alkyl durchführen.

Schema 5 zeigt ferner ein Verfahren zur Herstellung einer Verbindung der allgemeinen Formel I (Typ 19) mit R = $OR^1$, $R^1$ = H, S = U = H, T = H oder OH, V = $OR^7$, $R^7$ = H, W = Alkyl und insbesondere $C_{1-4}$-Alkyl, X = $CH_2OR^9$, $R^9$ = $COR^{10}$, $R^{10}$ = Alkyl und insbesondere $C_{1-6}$-Alkyl oder Alkenyl, Y = freies Elektronenpaar und Z = $CH_3$, bei dem man

(i) in einer ersten Stufe ein Verfahren durchführt und eine Verbindung (Typ 18) gewinnt und danach
(ii) in einer zweiten Stufe die angefallene Verbindung (Typ 18) in einem weiteren Verfahren zu einer Verbindung der allgemeinen Formel I mit den angegebenen Bedeutungen umsetzt und diese Verbindung gewinnt.

Schema 6 zeigt ein Verfahren zur Herstellung einer Verbindung der allgemeinen Formel I (Typ 20) mit R = $OR^1$, $R^1$ = Alkyl und insbesondere $C_{1-4}$-Alkyl oder Alkenyl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man eine Verbindung der allgemeinen Formel II (Typ 1, 2, 3, 4, 5 oder 6) oder ein Produkt eines Verfahrens (Typ 13) einer Alkylierung oder Alkenylierung unterwirft und eine Verbindung der allgemeinen Formel I mit den angegebenen Bedeutungen gewinnt.
Bei dem Verfahren kann man die Alkylierung oder Alkenylierung in Gegenwart von EDC, $R^1OH$ mit $R^1$ = Alkyl und insbesondere $C_{1-4}$-Alkyl oder Alkenyl und DMAP in Methylenchlorid durchführen.
Schema 6 zeigt ferner ein Verfahren zur Herstellung einer Verbindung der allgemeinen Formel I (Typ 21) mit R = $NHR^1$, NH-$NR^1R^2$, $NHOR^1$ oder $NH(CH_2)_{2-4}NR^1R^2$, $R^1$ und $R^2$ unabhängig voneinander = H, Alkyl und insbesondere $C_{1-6}$-Alkyl oder Aryl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man eine Verbindung der allgemeinen Formel II (Typ 1, 2, 3, 4, 5 oder 6) oder ein Produkt eines Verfahrens (Typ 13) mit einer Verbindung der Formel RH einer Aminierung unterwirft, wobei R die angegebenen Bedeutungen besitzt, und eine Verbindung der allgemeinen Formel I mit den angegebenen Bedeutungen gewinnt.
Bei dem Verfahren kann man die Umsetzung

(i) in Gegenwart von EDC in Methylenchlorid oder
(ii) in Gegenwart von i-Butylchlorformiat und Triethylamin in THF durchführen.

Schema 6 zeigt ferner ein Verfahren zur Herstellung einer Verbindung der allgemeinen Formel I (Typ 22) mit R = Alkyl und insbesondere $C_{1-4}$-Alkyl oder Alkenyl, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = $CH_3$, bei dem man eine Verbindung der allgemeinen Formel II (Typ 1, 2, 3, 4, 5 oder 6) oder ein Produkt eines Verfahrens (Typ 13) mit einer lithiumorganischen Verbindung der Formel RLi mit der angegebenen Bedeutung für R zu der Verbindung der allgemeinen Formel I mit den angegebenen Bedeutungen umsetzt.
Schema 6 zeigt ferner ein Verfahren zur Herstellung einer Verbindung der allgemeinen Formel I (Typ 23) mit R = Aminorest von 1-(2-Amino-$C_{2-4}$-alkyl)-pyrrol-2,5-dion, S = U = H, T = H oder $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl und insbesondere $C_{2-6}$-Alkenyl, Aryl oder Heteroaryl, Y = freies Elektronenpaar und Z = CH3, bei dem man eine Verbindung der allgemeinen Formel II (Typ 1, 2, 3, 4, 5 oder 6) oder ein Produkt eines Verfahrens (Typ 13) einer Aminierung mit 1-(2-Amino-$C_{2-4}$-alkyl)-pyrrol-2,5-dion unterwirft und die Verbindung der allgemeinen Formel I mit den angegebenen Bedeutungen gewinnt.
Bei dem Verfahren kann man die Aminierung in Gegenwart von EDC in Methylenchlorid durchführen.
Schema 7 zeigt ein Verfahren zur Herstellung einer Verbindung der allgemeinen Formel I (Typ 24) mit R = $OR^1$, $R^1$ = H, S = U = H, T = $OR^4$, $R^4$ = $P(O)\ (OR^6)_2$ mit $R^6$ = H oder Alkyl, insbesondere $C_{1-4}$-Alkyl, oder $R^4$ = $SO_3R^6$ mit $R^6$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Verbindung der allgemeinen Formel II (Typ 1, 2 oder 3) oder
(ii) ein Produkt eines Verfahrens (Typ 13) mit

(a) einer Verbindung der Formel $P(O)(OR^6)_2OH$ mit $R^6$ = H oder Alkyl und insbesondere $C_{1-4}$-Alkyl oder
(b) $SO_3$

umsetzt und die Verbindung der allgemeinen Formel I mit den angegebenen Bedeutungen gewinnt.
Bei dem Verfahren kann man die Variante (a) in Gegenwart von $I_2$ und Pyridin in Methylenchlorid durchführen.
Bei dem Verfahren Verfahren kann man die Variante (b) mit Pyridin-$SO_3$ durchführen.
Schema 7 zeigt ferner ein Verfahren zur Herstellung einer Verbindung der allgemeinen Formel I (Typ 25) mit R = $OR^1$, $R^1$ = H, S = U = H, T = $OR^4$, $R^4$ = $COR^5$, $R^5$ = Alkyl und insbesondere $C_{1-4}$-Alkyl, Alkenyl oder $N(R^{12})_2$, $R^{12}$

= Alkyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, bei dem man

(i) eine Verbindung der allgemeinen Formel II (Typ 1, 2 oder 3) oder

(ii) ein Produkt eines Verfahrens (Typ 13) einer Acylierung unterwirft und die Verbindung der allgemeinen Formel I mit den angegebenen Bedeutungen gewinnt.

Bei dem Verfahren kann man die Acylierung mit einem Acylhalogenid der Formel $R^5COCl$ mit $R^5$ = Alkyl und insbesondere $C_{1-4}$-Bkyl, Alkenyl oder $N(R^{12})_2$ und $R^{12}$ = Alkyl, insbesondere mit einem Acylchlorid, in Gegenwart einer organischen Base, insbesondere eines Trialkylamins, vorzugsweise Triethylamin, in einem organischen Lösungsmittel durchführen, insbesondere THF.

Schema 7 zeigt ferner ein Verfahren zur Herstellung einer Verbindung der allgemeinen Formel I (Typ 26) mit R = $OR^1$, $R^1$ = Alkyl und insbesondere $C_{1-4}$-Alkyl oder Alkenyl, S = U = H, T = $OR^4$, $R^4$ = Alkyl und insbesondere $C_{1-4}$-Alkyl oder Alkenyl, V = $OR^7$, $R^7$ = $COR^8$, $R^8$= Alkyl und insbesondere $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Verbindung der allgemeinen Formel II (Typ 1, 2 oder 3) oder

(ii) ein Produkt eines Verfahrens (Typ 13) einer Alkylierung unterwirft und die Verbindung der allgemeinen Formel gemäß Anspruch 1 mit den angegebenen Bedeutungen gewinnt.

Bei dem Verfahren kann man mit einem Alkyliodid der Formel $R^4I$ mit $R^4$ = Alkyl und insbesondere $C_{1-4}$-Alkyl oder Alkenyl in Gegenwart einer schwachen Base, insbesondere $Ag_2O$, in einem organischen Lösungsmittel alkylieren, insbesondere Methylenchlorid.

Bei dem Verfahren kann man mit Diazomethan in einem organischen Lösungsmittel methylieren, insbesondere Methanol.

Schema 7 zeigt ferner ein Verfahren zur Herstellung einer Verbindung der allgemeinen Formel I (Typ 27) mit R = $OR^1$, $R^1$ = H, S = U = H, T = $OR^4$, $R^4$ = Alkyl und insbesondere $C_{1-4}$-Alkyl oder Alkenyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt eines Verfahrens (Typ 26) enzymatisch einer partiellen Dealkylierung oder Dealkenylierung unterwirft und die Verbindung der allgemeinen Formel I mit den angegebenen Bedeutungen gewinnt.

Bei dem Verfahren kann man als Enzym eine Esterase verwenden, insbesondere Schweineleber-Esterase.

Schema 7 zeigt ferner ein Verfahren zur Herstellung einer Verbindung der allgemeinen Formel I (Typ 27) R = $OR^1$, $R^1$ = H, S = U = H, T = $OR^4$, $R^4$ = Alkyl und insbesondere $C_{1-4}$-Alkyl oder Alkenyl, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = $C_{1-6}$-Alkyl, Alkenyl, insbesondere $C_{2-6}$-Alkenyl, Aryl oder Heteroaryl, bei dem man

(a) in einer ersten Stufe

(i) eine Verbindung der allgemeinen Formel II (Typ 1, 2 oder 3) oder
(ii) ein Produkt eines Verfahrens (Typ 13) einem Verfahren unterwirft und eine erfindungsgemäße Verbindung (Typ 26) gewinnt und

(b) in einer zweiten Stufe die angefallene erfindungsgemäße Verbindung (Typ 26) einem weiteren Verfahren zu einer Verbindung der allgemeinen Formel I mit den angegebenen Bedeutungen umsetzt und diese Verbindung gewinnt.

Schema 8 ein Verfahren zur Herstellung einer Verbindung der allgemeinen Formel I (Typ 28 und ggf. 29) mit R = $OR^1$, $R^1$ = H, S = H oder Hal, T = $OR^4$, $R^4$ = H, U = Hal, V = $OR^7$, $R^7$ = $COR^8$, $R^8$ = Alkyl und insbesondere $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, bei dem man

(i) eine Verbindung der allgemeinen Formel II (Typ 1, 2, 3, 4, 5 oder 6) oder
(ii) ein Produkt eines Verfahrens (Typ 13) einer Halogenierung oder Dihalogenierung in ortho-Stellung zum T-Substituenten unterwirft und die Verbindung der allgemeinen Formel I mit den angegebenen Bedeutungen gewinnt.

Bei dem Verfahren kann man die Halogenierung in Gegenwart von $C_5Cl_5$NF-triflat, $SO_2Cl_2$, NBS und ICl durchführen.

Schema 8 zeigt ferner ein Verfahren zur Herstellung einer Verbindung der allgemeinen Formel I (Typ 30) mit R = $OR^1$, $R^1$ = H, S = H, T = $OR^4$, $R^4$ = H, U = $NO^2$, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(i) eine Verbindung der allgemeinen Formel II (Typ 1, 2, 3, 4, 5 oder 6) oder
(ii) ein Produkt eines Verfahrens (Typ 13) einer Nitrierung in ortho-Stellung zum T-Substituenten unterwirft und die Verbindung der allgemeinen Formel I mit den angegebenen Bedeutungen gewinnt.

Bei dem Verfahren kann man die Nitrierung mit einem Alkalimetallnitrit, insbesondere Natriumnitrit, und Essigsäure in Gegenwart eines organischen Lösungsmittels durchführen, insbesondere Ethanol.

Schema 8 zeigt ferner ein Verfahren zur Herstellung einer Verbindung der allgemeinen Formel I (Typ 31) mit R = $OR^1$, $R^1$ = H, S = H, T = $OR^4$, $R^4$ = H, U = $NH_2$, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt eines Verfahrens (Typ 30) einer katalytischen Reduktion unterwirft und die Verbindung der allgemeinen Formel I mit den angegebenen Bedeutungen gewinnt.

Bei dem Verfahren kann man mit elementarem Wasserstoff in Gegenwart von Palladium/Aktivkohle reduzieren, insbesondere in einem organischen Lösungsmittel, vorzugsweise Ethanol.

Schema 8 ein Verfahren zur Herstellung einer Verbindung der allgemeinen Formel I (Typ 31) mit R = $OR^1$, $R^1$ = H, S = H, T = $OR^4$, $R^4$ = H, U = $NH_2$, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man

(a) in einer ersten Stufe

(i) eine Verbindung der allgemeinen Formel II (Typ 1, 2, 3, 4, 5 oder 6) oder
(ii) ein Produkt eines Verfahrens (Typ 13) einem weiteren Verfahren unterwirft und eine Verbindung (Typ 30) gewinnt und

(b) in einer zweiten Stufe das erhaltene Produkt (Typ 30) einem weiteren Verfahren unterwirft und die Verbindung der allgemeinen Formel I mit den angegebenen Bedeutungen gewinnt.

Schema 8 ein Verfahren zur Herstellung einer Verbindung der allgemeinen Formel I (Typ 32) mit R = $OR^1$, $R^1$ = H, S = H, T = $OR^4$, $R^4$ = H, U = $NHR^3$, $R^3$ = Alkyl-CO und insbesondere $C_{1-4}$-Alkyl-CO, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar und Z = $CH_3$, bei dem man ein Produkt eines Verfahrens (Typ 31) einer Alkylierung unterwirft und die Verbindung der allgemeinen Formel I mit den angegebenen Bedeutungen gewinnt.

Bei dem Verfahren kann man mit einem Säureanhydrid der Formel $(R^3)_2O$ mit $R^3$ = CO-$C_{1-4}$-Alkyl alkylieren.

Schema 8 zeigt ferner ein Verfahren zur Herstellung einer Verbindung der allgemeinen Formel I (Typ 32) mit R = $OR^1$, $R^1$ = H, S = H, T = $OR^4$, $R^4$ = H, U = $NHR^3$, $R^3$ = Alkyl-CO und insbesondere $C_{1-4}$-Alkyl-CO, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl, vorzugsweise $C_{1-6}$-Alkyl, Alkenyl, insbesondere $C_{2-6}$-Alkenyl, Aryl oder Heteroaryl, bei dem man

(a) in einer fakultativen ersten Stufe

(i) eine Verbindung der allgemeinen Formel II (Typ 1, 2, 3, 4, 5 oder 6) oder
(ii) ein Produkt eines Verfahrens (Typ 13) einem weiteren Verfahrens unterwirft,

(b) in einer zweiten Stufe das erhaltene Produkt (Typ 30) einem weiteren Verfahren unterwirft und
(c) in einer dritten Stufe die angefallene Verbindung (Typ 31) einem weiteren Verfahren unterwirft und

die Verbindung der allgemeinen Formel I mit den angegebenen Bedeutungen gewinnt.

Schema 9 zeigt ein Verfahren zur Herstellung einer Verbindung der allgemeinen Formel I (Typ 33) mit R = $OR^1$, $R^1$ = H, S = U = H, T = $OR^4$, $R^4$ = H, V = $OR^7$, $R^7$ = $COR^8$, $R^8$ = Alkyl, vorzugsweise $C_{1-4}$-Alkyl, insbesondere

Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = O und Z = $CH_3$, bei dem man

(i) eine Verbindung der allgemeinen Formel II (Typ 1, 2, 3, 4, 5 oder 6) oder
(ii) ein Produkt eines Verfahrens (Typ 13) einer Reaktion zur Bildung eines N-Oxids unterwirft und die Verbindung der allgemeinen Formel I mit den angegebenen Bedeutungen gewinnt.

Bei dem Verfahren kann man die N-Oxid-Bildung mit m-CPBA in einem organischen Lösungsmittel durchführen, insbesondere Methylenchlorid.

Schema 9 zeigt ferner ein Verfahren zur Herstellung einer Verbindung der allgemeinen Formel I (Typ 34) mit R = $OR^1$, $R^1$ = H, S = U = H, T = $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar, Z = $COR^{11}$ und $R^{11}$ = Alkyl, vorzugsweise $C_{1-4}$-Alkyl, insbesondere Methyl, bei dem man das Produkt eines Verfahrens (Typ 33) mit einem Acylierungsmittel umsetzt und die Verbindung der allgemeinen Formel I mit den angegebenen Bedeutungen gewinnt.

Bei dem Verfahren kann man die Acylierung mit einem Säureanhydrid durchführen, insbesondere Essigsäureanhydrid, vorzugsweise bei erhöhter Temperatur.

Schema 9 zeigt ferner ein Verfahren zur Herstellung einer Verbindung der allgemeinen Formel I (Typ 34) mit R = $OR^1$, $R^1$ = H, S = U = H, T = $OR^4$, $R^4$ = H, V = $OR^7$, $R^7$ = $COR^8$, $R^8$ = Alkyl, vorzugsweise $C_{1-4}$-Alkyl, insbesondere Methyl, W = H, X = $CH_2OR^9$, $R^9$ = $COR^{10}$, $R^{10}$ = Alkyl und insbesondere $C_{1-6}$-Alkyl, Alkenyl, insbesondere $C_{2-6}$-Alkenyl, Aryl oder Heteroaryl, Y = freies Elektronenpaar, Z = $COR^{11}$ und $R^{11}$ = Alkyl, vorzugsweise $C_{1-4}$-Alkyl, insbesondere Methyl, bei dem man

(a) in einer ersten Stufe

(i) eine Verbindung der allgemeinen Formel II (Typ 1, 2, 3, 4, 5 oder 6) oder
(ii) ein Produkt eines Verfahrens (Typ 13) einem Verfahren unterwirft und

(b) in einer zweiten Stufe das erhaltene Produkt (Typ 33) einem weiteren Verfahren unterwirft und die Verbindung der allgemeinen Formel gemäß Anspruch 1 mit den angegebenen Bedeutungen gewinnt.

Eine weitere Ausführungsform der Erfindung betrifft ein therapeutisches Mittel, insbesondere Cytostatikum, mit einer oder mehreren erfindungsgemäßen Verbindungen als Wirkstoff neben einem oder mehreren fakultativen üblichen Trägern und/oder einem oder mehreren fakultativen üblichen Verdünnungsmitteln.

Schließlich betrifft eine Ausführungsform der Erfindung ein therapeutisches Mittel, insbesondere Cytostatikum, mit einem oder mehreren Produkten eines erfindungsgemäßen Verfahrens als Wirkstoff neben einem oder mehreren fakultativen üblichen Trägern und/oder einem oder mehreren fakultativen üblichen Verdünnungsmitteln.

Nachstehend wird die Erfindung durch Beispiele näher erläutert.

*Tubulysin-Derivat **7a:** $R^1$ = OH (Schema 1)*

9.9 mg (11.7 µmol) Tubulysin A **(1)** wurden in 200 µl Dioxan gelöst und mit 1 ml 0.1 M Salzsäure-Lösung versetzt. Der Reaktionsansatz wurde bei 50°C 8 h gerührt. Anschließend wurde die Mischung liophilisiert und der Rückstand mittels präp. HPLC ($CH_3CN/H_2O$ 35/65 mit 50 mM $NH_4Ac$, pH = 6.5), wobei 5.3 mg (59 %) **7a** erhalten wurden.

**$R_f$** 0.55; $[\alpha]^{22}_D$ -7.0 (c 0.89-MeOH) ; **UV** (MeOH): $\lambda_{max}$ nm (lgε) 226 (4.13), 250 (3.91); **IR** (KBr): $\nu_{max}$ 3386, 2963, 2934, 1655, 1546, 1232 $cm^{-1}$; **$^1H$ NMR** (DMSO-$D_6$ 600 MHz): wie Tubulysin A (1)außer Tuv δ 8.06 (1H, s, H-3), 6.18 (1H, d, J = 11.9 Hz, H-11b), 5.37 (1H, d, J = 11.8 Hz, H-11a), 4.63 (1H, br, H-5), 4.10 (1H, br, H-7), 2.20 (1H, m, H-6b), 1.99 (1H, m, H-8), 1.98 (1H, m, H-6a), 1.91 (1H, m, H-2'b), 1.48 (1H, m, H-3'b), 1.44 (1H, m, H-3'a), 1.42 (1H, m, H-2'a), 0.92 (3H, d, J = 6.4 Hz, H-9), 0.80 (3H, t, J = 7.3 Hz, H-4'), 0.73 (3H, d, J = 6.2 Hz, H-10); **$^{13}C$ NMR** (DMSO-$D_6$ 150 MHz): wie Tubulysin A (1), außer Tuv δ 178.0 (s, C-4), 174.4 (s, C-1'), 160.0 (s, C-1), 149.6 (s, C-2), 123.0 (d, C-3), 68.0 (t, C-11), 67.5 (d, C-5), 55.0 (d, C-7), 37.4 (t, C-2'), 35.7 (t, C-6), 30.6 (d, C-8), 20.1 (q, C-9), 19.5 (q, C-10), 17.7 (t, C-3'), 13.3 (q, C-4'); **DCI MS:** m/z $[M+H^+]$ 760 (4); **HRMS (DCI):** $C_{38}H_{58}N_5O_9S$: 760.3917 $[M+H]^+$ (ber.: 760.3955).

*Tubulysin-Derivat **8a:** $R^1$ = OH (Schema 1)*

20.0 mg (23.7 µmol) Tubuylsin A **(1)** wurden mit 500 µl 0.1 M Salzsäure versetzt. Der Reaktionsansatz wurde 5 Minuten bei 100°C gerührt, anschließend abgekühlt und mit gesättigter $NaHCO_3$-Lösung neutralisiert (pH = 7). Nachdem dreimal mit Ethylacetat extrahiert wurde, wurden die vereinigten organischen Phasen eingeengt. Das Rohprodukt

wurde mittels präp. HPLC ($CH_3CN/H_2O$ 35/65 mit 50 mM $NH_4Ac$, pH = 6.5) gereinigt, wobei 6.4 mg (37 %) **8a,** 2.7 mg (15 %) **7a** und 5.1 mg (31 %) **10a** erhalten wurden.

**8a:**

**$R_f$** 0.55 ; $[\alpha]^{22}_D$ -10.2 (c 1.0 MeOH); **UV** (MeOH) : $\lambda_{max}$ nm (lgε) 225 (4.10), 250 (3.94); **IR** (KBr) $\nu_{max}$ 3389, 3251, 2962, 2934, 1658, 1547, 1228 $cm^{-1}$; **$^1$H NMR** (DMSO-$D_6$ 600 MHz): wie Tubulysin A **(1)** außer Tuv δ 8.17 (1H, s, H-3), 7.92 (1H, br, NH-7), 5.76 (1H, dd, J = 10.5, 3.0 Hz, H-5), 3.86 (1H, m, H-7), 2.13 (1H, m, H-6b), 2.09 (3H, s, H-5OAc), 1.95 (1H, m, H-6a), 1.73 (1H, m, H-8), 0.84 (3H, d, J = 6.4 Hz, H-10), 0.83 (3H, d, J = 6.0 Hz, H-9), Ile δ 7.54 (1H, d, J = 9.3 Hz, NH-2), 4.18 (1H, dd, J = 9.1 Hz, H-2), 1.75 (1H, m, H-3), 1.48 (1H, m, H-4b), 1.07 (1H, m, H-4a), 0.85 (3H, m, H-6), 0.81 (3H, m, H-5); **$^{13}$C NMR** (DMSO-$D_6$ 150 MHz): wie Tubulysin A (1) außer Tuv δ 169.6 (s, C-5OAc), 169.6 (s, C-4), 159.8 (s, C-1), 149.8 (s, C-2), 124.0 (d, C-3), 69.5 (d, C-5), 49.5 (d, C-7), 36.4 (t, C-6), 31.7 (d, C-8), 20.6 (q, C-5OAc), 18.9 (q, C-9), 18.0 (q, C-10), Ile δ 171.1 (s, C-1), 56.7 (d, C-2), 36.2 (d, C-3), 24.3 (t, C-4), 15.6 (q, C-6), 10.6 (q, C-5); **DCI MS** m/z $[M+H^+]$ 730 (100), 672 (15); **HRMS (DCI):** $C_{37}H_{56}N_5O_8S$: 730.3839 $[M+H]^+$ (ber.: 730.3850).

*Tubulysin Derivat **9a:** R = i-$C_4H_9$, $R^1$ = OH (Schema 1)*

9.6 mg (11.4 μmol) Tubulysin A **(1)** wurden in 1 ml Methanol gelöst und in Abständen von drei Stunden mit jeweils 10 μl (133.6 μmol) 25 % Ammoniak versetzt und bei Raumtemperatur gerührt. Anschließend wurde der Reaktionsansatz mit 18 % Salzsäure auf pH 5 eingestellt und dreimal mit Ethylacetat extrahiert. Die vereinigten organischen Phasen wurden eingeengt und mittels PSC ($CH_2Cl_2$/MeOH 85/15) gereinigt. Es wurden 2.5 mg (27 %) **9a,** 2.3 mg (29 %) **10a** und 1.7 mg (18 %) **1** isoliert.

**9a:**

**ESI MS** (1 eV): 802 $[M+H]^+$; **$^1$H-NMR** (DMSO-$D_6$, 600 MHz): δ = 4.63 (br, 1H, H-5), 4.10 (br, 1H, H-7), 2.20 (m, 1H, H-6b), 1.99 (m, 1H, H-8), 1.98 (m, 1H, H-6a)

*Tubulysin-Derivat **10a:** $R^1$ = OH (Schema 1)*

5.4 mg (6.8 μmol) Tubulysin A **(1)** wurden in 300 μl Methanol gelöst, mit 67.0 μl 1 M Natronlauge (67.6 μmol) versetzt und 15 min bei Raumtemperatur gerührt. Anschließend wurde der Reaktionsansatz mit Wasser verdünnt und mit 1 M Salzsäurelösung auf pH 7 eingestellt. Nach dreimaliger Extraktion mit Ethylacetat wurden die vereinigten organischen Phasen eingeengt. Der Rückstand wurde mittels präp. HPLC ($CH_3CN/H_2O$ 35/65 mit 50 mM $NH_4Ac$, pH = 6.5) gereinigt, wobei 2.5 mg (57 %) **10a** erhalten wurden.

**$R_f$** 0.40; $[\alpha]^{22}_D$ -3.0 (c 0.66 MeOH); **UV** (MeOH) : $\lambda_{max}$ nm (lg ε) 225 (4.12), 250 (3.92); **IR** (KBr): $\nu_{max}$ 3376 $cm^{-1}$, 3285, 2960, 2929, 1656, 1547; **$^1$H NMR** (DMSO-$D_6$ 600 MHz): wie Tubulysin A **(1)** außer Tuv δ 8.06 (1H, s, H-3), 7.67 (1H, br, NH-7), 4.65 (1H, ddbr, J = 8.7 Hz, H-5), 3.97 (1H, m, H-7), 1.98 (1H, m, H-6b), 1.79 (1H, m, H-6a), 1.74 (1H, m, H-8), 0.85 (3H, d, J = 6.7 Hz, H-9), 0.84 (3H, d, J = 6.2 Hz, H-10), Ile δ 7.75 (1H, br, NH-2), 4.15 (1H, dd, J = 8.6 Hz, H-2), 1.81 (1H, m, H-3), 1.55 (1H, m, H-4b), 1.11 (1H, m, H-4a), 0.86 (3H, d, J = 6.4 Hz, H-6), 0.80 (3H, t, J = 7.2 Hz, H-5); **$^{13}$C NMR** (DMSO-$D_6$ 150 MHz): wie Tubulysin A **(1)** außer Tuv δ 177.9 (s, C-4), 160.1 (s, C-1), 149.7 (s, C-2), 122.8 (d, C-3), 67.9 (d, C-5), 50.3 (d, C-7), 40.5 (t, C-6), 31.8 (d, C-8), 19.0 (q, C-9), 18.1 (q, C-10), Ile δ 171.4 (s, C-1), 57.1 (d, C-2), 36.2 (d, C-3), 24.6 (t, C-4), 15.6 (q, C-6), 10.4 (q, C-5); **DCI MS:** m/z $[M+H^+]$ 688(100), 256 (12), 223 (6), 98 (4); **HRMS (DCI):** $C_{35}H_{54}N_5O_7S$: 688.3799 $[M+H]^+$ (ber.: 688.3744).

*Methylester von Tubulysin-Derivat **10a:***

2.5 mg (3.6 μmol) 10a wurden in 200 μl Methanol gelöst, mit etherischer Diazomethanlösung versetzt und 10 min bei Raumtemperatur gerührt. Die Reinigung erfolgte direkt über eine PSC ($CH_2Cl_2$/MeOH 85/15) und ergab 2.8 mg (62 %) Methylester von **10a.**

**$R_f$** ($CH_2Cl_2$/MeOH 85/15): 0.32; **IR** (KBr): $\tilde{\nu}$ = 3380 $cm^{-1}$ (m), 2960 (s), 2931 (s), 2872 (w), 1743 (s), 1659 (vs), 1516 (m), 1371 (w), 1229 (s), 1092 (w); **UV** (MeOH): $\lambda_{max}$ (lg ε) = 204 nm (4.53), 226 (4.27), 244 (sh, 4.01), 276 (sh, 3.38); **DCI MS** (120 eV, $NH_3$): 702 $[M+H]^+$; **$^1$H-NMR** ($CD_3OD$, 300 MHz): δ = 1.18 (d, 3 H, Tut10-H), 3.64 (s, 3 H, Tut11-H).

*Cyclo-Tubulysin A **(11a):** $R^1$ = OH (Schema 1)*

9.6 mg (11.3 μmol) Tubulysin A **(1),** verteilt als dünner Film an den Glaswandungen des Reaktions-gefäßes

wurde mit 1 ml 0.5 M Salzsäure-Lösung versetzt und 30 min bei 100°C gerührt. Der Reaktionsansatz wurde anschließend lyophilisiert und der Rückstand über PSC ($CH_2Cl_2$/MeOH 90/10) gereinigt, wobei 3.9 mg (50 %) **11a** und 1.6 mg (21 %) **10a** erhalten wurden.

**11a:**

**ESI MS** (1 eV): 699 $[M+H]^+$; **$^1$H-NMR** (DMSO-$D_6$, 300 MHz): $\delta$ = 8.15 (s, 1 H, Tuvt3-H), 5.32 (d, 1 H, Tuv5-H), 1.7 (m, 1 H, Tuv6a-H), 2.3 (m, 1 H, Tuv6b-H), 4.32 (m, 1 H, Tuv7-H), 1.8 (m, 1 H, Tuv8-H), 0.75 (d, 3 H, Tuv9-$H_3$), 0.95 (d, 3 H, Tuv10-$H_3$), 4.86 (d, 1 H, Tuv11a-H), 5.65 (d, 1 H, Tuv11b-H).

*Tubulysin-Derivate **12 und 13***

Man kann aus einem Tubulysin-Derivat 7a ein Tubulysin-Derivat 12 herstellen, indem man 7a mit Acetylchlorid in Triethylamin umsetzt.

Geht man von einem Tubulysin-Derivat Typ 7 mit T = $OR^4$, $R^4$ = $COR^5$ und $R^5$ = Methyl oder Ethyl aus, so kann man das anfallende Tybulysin-Derivat Typ 12 mit Ammoniak zu einem Tubulysin-Derivat Typ 13 verseifen.

*Tubulysin A-methylether **(14a):** $R^1$ = OH (Schema 3)*

Zu einer Lösung von 10.0 mg (11.9 µmol) Tubulysin A **(1)** in 500 µl abs. THF wurden 500 µl abs. Ethanol und 1 mg (5.3 µmol) *p*-Toluolsulfonsäure zugegeben. Der Reaktionsansatz wurde 20 Minuten bei 80°C gerührt. Anschließend wurde vom Lösungsmittel befreit und das Rohprodukt mittels PSC ($CH_2Cl_2$/MeOH 90/10) gereinigt. Es wurden 3.1 mg (33 %) **14a** erhalten.

**ESI MS** (1 eV): 788 $[M+H]^+$

*Tubulysin-Derivat **15***

Man kann ein Tubulysin-Derivat 15 dadurch herstellen, daß man ein Tubulysin-Derivat 7a mit $NaCNBH_3$ und TFA in Methanol reduziert.

*Tubulysin-Derivat **16***

Man kann ein Tubulysin-Derivat 16 dadurch herstellen, daß man ein Tubulysin-Derivat 9a mit Acetylchlorid in Triethylamin acetyliert.

*Tubulysin-Derivat **17***

Man kann ein Tubulysin-Derivat 17 dadurch herstellen, daß man ein Tubulysin-Derivat 9a in Gegenwart von $CH_3COOH$ bzw. DAST katalytisch an einem Pd/C-Katalysator mit elementarem Wasserstoff hydriert.

*Tubulysin-Derivate **18 und 19***

Man kann ein Tubulysin-Dervat 18 dadurch herstellen, daß man ein Tybulysin-Derivat 9a in Gegenwart von TPAP und NMO oxidiert.

Das erhaltene Tubulysin-Derivat 18 kann man mit Ethylmagnesiumbromid zu einem Tubulysin-Derivat 19 umsetzen.

*Tubulysin A-methylester **(20a):** $R^2$ = $CH_3$ (Schema 6)*

19.0 mg (22.5 µmol)Tubulysin A **(1)** wurden in 300 µl Methanol gelöst und bei Raumtemperatur zweimal in Abständen von 15 Minuten mit etherischer Diazomethanlösung versetzt. Der Reaktionsansatz wurde eingeengt, die Reinigung des Rohproduktes erfolgte über PSC ($CH_2Cl_2$/MeOH 90/10) wobei 11.7 mg (61 %) **20a** erhalten wurden.

**IR** (KBr): $\tilde{\nu}$ = 3383 $cm^{-1}$ (m), 2962 (s), 2875 (w), 1739 (vs), 1666 (vs), 1516 (s), 1227 (vs), 1091 (w); UV (MeOH): $\lambda_{max}$ (lg $\varepsilon$) = 203 nm (4.61), 225 (4.34), 243 (sh, 4.11), 276 (sh, 3.41); DCI MS (120 eV, *I*-Butan): 858 $[M+H]^+$; **$^1$H-NMR** (DMSO-$D_6$, 300 MHz): $\delta$ = 2.43 (m, 1 H, Tut2-H), 1.06 (d, 3 H, Tut10-H), 3.53 (s, 3 H, Tut11-H); **$^{13}$C-NMR** (DMSO-$D_6$, 75 MHz): $\delta$ = 175.8 (TutC1), 35.8 (TutC2), 17.6 (TutC10), 51.3 (TutC11).

*Tubulysin-A-ethylester **(20b):** $R^1$ = OH, $R^2$ = $C_2H_5$ (Schema 6)*

Zu einer Lösung aus 5.2 mg (6.2 μmol) Tubulysin A **(1)** in 300 μl Dichlormethan wurden13.5 μl (9.3 μmol) Ethanol, 1.8 mg (9.3 μmol) EDC und 57 μl (9.3 μmol) einer DMAP-Lösung (5 mg/250 μl $CH_2Cl_2$) gegeben. Der Reaktionsansatz wurde bei Raumtemperatur über Nacht gerührt. Anschließend erfolgte eine Reinigung der Rohprodukte mittels PSC ($CH_2Cl_2$/MeOH 90/10), wobei 1.7 mg (32 %) **20b,** 0.7 mg (13 %) **25a** und 1.0 mg (18 %) **20b** mit $R^1$ = $OCOCH_3$ erhalten wurden.

**20b:**

**DCI MS** (120 eV, $NH_3$): 872 $[M+H]^+$; **HRMS (DCI)**: $C_{45}H_{69}N_5O_{10}S$: $[M+H]^+$ ber.: 872.4843 (gef.: 872.4917); **$^1$H-NMR** ($CD_3OD$, 300 MHz): δ = 2.58 (m, 1 H, Tut2-H), 1.19 (d, 3 H, Tut10-H), 4.11 (q, 2 H, Tut11-H), 1.23 (t, 3 H, Tut12-H).

**20b** mit $R^1$ = $OCOCH_3$

**$R_f$** ($CH_2Cl_2$/MeOH 90/10): 0.51; **IR** (KBr): $\tilde{\nu}$ = 3392 $cm^{-1}$ (m), 2962 (s), 2920 (s), 2874 (w), 1736 (s), 1667 (vs), 1507 (m), 1370 (w), 1218 (s), 1195 (s); **UV** (MeOH): $\lambda_{max}$ (lg ε) = 203 nm (4.60), 218 (4.30), 227 (sh, 4.23), 248 (sh, 3.98); **DCI MS** (120 eV, $NH_3$): 914 $[M+H]^+$; **HRMS (DCI)**: $C_{47}H_{71}N_5O_{11}S$: $[M+H]^+$ ber.: 814.4949 gef.: 914.5044; **$^1$H-NMR** ($CD_3OD$, 300 MHz): δ = 7.29 (d, 2 H, Tut7-H), 7.02 (d, 2 H, Tut8-H), 1.20 (d, 3 H, Tut10-H), 4.11 (q, 2 H, Tut11-H), 1.23 (t, 3 H, Tut12-H), 2.28 (s, 3-H, Tut13-H).

*Tubulysin A-propylester **(20c):** $R^1$ = OH, $R^2$ = $C_3H_7$ (Schema 6)*

Eine Lösung aus 11.3 mg (13.4 μmol) Tubulysin A **(1)** in 450 μl Dichlormethan/Diethylether (1/2) wurde mit 6.5 μl (67.0 μmol) Propyliodid und 6.2 mg (26.8 μmol) Silber(I)oxid versetzt. Der Reaktionsansatz wurde über Nacht bei. Raumtemperatur gerührt, anschließend über Celite filtriert und der Rückstand mit Dichlormethan gewaschen. Die vereinigten organischen Phasen wurden eingeengt und mittels PSC ($CH_2Cl_2$/MeOH 90/10) gereinigt, wobei 7.6 mg (64 %) **20c** erhalten wurden.

**IR** (KBr): $\tilde{\nu}$ = 3387 $cm^{-1}$ (m), 2964 (s), 2938 (m), 2876 (w), 1737 (s), 1667 (vs), 1516 (s), 1416 (m), 1370 (w), 1225 (s), 1094 (w); **UV** (MeOH): $\lambda_{max}$ (lg ε) = 204 nm (4.64), 224 (4.39), 246 (sh, 4.12), 276 (3.60); **DCI MS** (120 eV, $NH_3$): 886 $[M+H]^+$; **$^1$H-NMR** ($CD_3OD$, 300 MHz): δ = 2.63 (m, 1 H, Tut2-H), 1.68 (m, 1 H, Tut3a-H), 2.03 (m, 1 H, Tut3b-H), 4.32 (m, 2 H, Tut4-H), 2.83 (m, 2 H, Tut5-H), 6.72 (d, 1 H, Tut7-H), 7.08 (d, 1 H, Tut8-H), 1.20 (d, 3 H, Tut10-H), 4.02 (t, 2 H, Tut11-H), 1.64 (m, 2 H, Tut12-H), 0.95 (t, 3 H, Tut13-H); **$^{13}$C-NMR** ($CD_3OD$, 75 MHz): δ = 177.9 (TutC1), 38.1 (TutC2), 38.9 (TutC3), 50.7 (TutC4), 41.5 (TutC5), 130.0 (TutC6), 116.2 (TutC7), 131.4 (TutC8), 157.1 (TutC9), 18.4 (TutC10), 67.2 (TutC11), 23.0 (TutC12).10.7 (TutC13).

*Tubulysin-A-propylamid **(21a)**: $R^1$ = OH, $R^2$ = $C_3H_7$ (Schema 6)*

Zu einer Lösung aus 4.9 mg (5.8 μmol) Tubulysin A (1) in 300 μl Dichlormethan wurden 180 μl (19.2 μmol) EDC-Lösung (4 mg/200 μl $CH_2Cl_2$) und 36 μl (43.5 μmol) Propylamin-Lösung (10 μl/100 μl $CH_2Cl_2$) gegeben. Der Reaktionsansatz wurde zwei Tage bei Raumtemperatur gerührt. Die Reinigung erfolgte mittels PSC ($CH_2Cl_2$/MeOH 90/10) und ergab 1.0 mg (20 %) 21a.

**ESI** MS (1 eV): 885 $[M+H]^+$; **$^1$H-NMR** ($CD_3OD$, 300 MHz): δ = 2.49 (m, 1 H, Tut2-H), 1.14 (d, 3 H, Tut10-H), 3.15 (t, 2 H, Tut11-H), 1.56 (m, 2 H, Tut12-H), 0.96 (t, 3 H, Tut13-H).

*Tubulysin-A-hexylamid **(21b)**: $R^1$ = OH, $R^2$ = $C_6H_{13}$ (Schema 6)*

2.8 μl (16.5 μmol) Hünig-Base wurden in 200 μl abs. THF bei 0˚C gelöst und mit 1.4 μl (11.0 μmol) *i*-Butylchlorformiat versetzt. Nach 5 min wurden 9.3 mg (11.0 μmol) Tubulysin A (1), gelöst in 300 μl abs. THF, zugegeben und weitere 40 min bei 0˚C gerührt. Anschließend wurde der Reaktionsansatz mit 1.6 μl (12.1 μmol) Hexylamin und 2.8 μl (16.5 μmol) Hünig-Base versetzt und bei Raumtemperatur über Nacht gerührt. Eine Reinigung des Rohproduktes erfolgte direkt mittels PSC ($CH_2Cl_2$/MeOH 90/10) und lieferte neben 6.0 mg (65 %) 1, 3.6 mg (35 %) 21b.

**$R_f$** ($CH_2Cl_2$/MeOH 90/10): 0.41; IR (KBr): $\tilde{\nu}$ = 3389 $cm^{-1}$ (m), 2960 (s), 2932 (s), 2872 (w), 1743 (m), 1654 (vs), 1516 (m), 1418 (m), 1228 (s); **UV** (MeOH): $\lambda_{max}$ (lg ε) = 204 nm (4.62), 226 (4.31), 242 (sh, 4.09), 278 (sh, 3.41); DCI MS (120 eV, $NH_3$): 927 $[M+H]^+$; **HRMS (DCI):** $C_{49}H_{78}N_6O_9S$: $[M+H]^+$ ber.: 927.5629 (gef.: 927.5641).

*Tubulysin-A-benzylamid* ***(21c):*** *$R^1$ = OH, $R^2$ = $CH_2C_6H_5$ (Schema 6)*

4.5 µl (26.8 µmol) Hünig-Base wurden in 200 µl abs. THF gelöst und auf 0°C gekühlt. Die Lösung wurde mit 2.4 µl (17.9 µmol) Chlorameisensäureisobutylester versetzt und 5 min gerührt. Anschließend wurde eine Lösung von 10 mg (11.9 µmol) Tubulysin A in 300 µl abs. THF zugegeben und bei 0°C gerührt. Nach 30 min wurden 1.4 µl (13.1 µmol) Benzylamin und 3 µl (17.9 µmol) Hünig-Base zugegeben und das Reaktionsgemisch über Nacht bei Raumtemperatur gerührt. Der Reaktionsansatz wurde direkt mittels PSC ($CH_2Cl_2$/Methanol 90/10) gereinigt, wobei 3.5 mg (37 %) **NT19** erhalten wurden.

**$R_f$** ($CH_2Cl_2$/MeOH 90/10): 0.40; **Drehwert:** $[\alpha]_D^{20}$ = + 27.2 (c 0.22, Methanol); **IR** (KBr): $\tilde{\nu}$ = 3383 $cm^{-1}$ (m), 2962 (m), 2935 (m), 2875 (w), 1742 (m), 1661 (vs), 1516 (m), 1420 (w), 1371 (w), 1227 (s), 1094 (w); **UV** (MeOH): $\lambda_{max}$ (lg ε) = 204 nm (4.62), 224 (sh, 4.24), 246 (sh, 3.94), 277 (sh, 3.24); **DCI MS** (120 eV, $NH_3$): $[M+H]^+$; **ESI MS** (1 eV): 932 $[M+H]^+$; **$^1$H-NMR** ($CD_3OD$, 300 MHz): δ = 2.54 (m, 1 H, Tut2-H), 1.64 (m, 1 H, Tut3a-H), 2.03 (m, 1 H, Tut3b-H), 4.24 (m, 1 H, Tut4-H), 2.82 (bd, 2 H, Tut5-H), 7.01 (d, 2 H, Tut7-H), 6.69 (d, 2 H, Tut8-H), 1.17 (d, 3 H, Tut10-H), 4.42 (dd, 2 H, Tut11-H), 7.2-7.4 (m, 5 H, Tut13,14,15-H); **$^{13}$C-NMR** ($CD_3OD$, 75 MHz): δ = 187.5 (TutC1), 39.1 (TutC2), 40.3 (TutC3), 51.3 (TutC4), 41.3 (TutC5), 130.0 (TutC6), 131.4 (TutC7), 116.2 (TutC8), 157.0 (TutC9), 19.1 (TutC10), 44.2 (TutC11), 140.2 (TutC12), 128.7 (TutC13), 129.5 . (TutC14), 128.1 (TutC15).

*Tubulysin-Derivat* ***22***

Ein Tubulysin-Derivat 22 kann man dadurch gewinnen, daß man Tubulysin 1 mit Methyl- oder Ethyllithium zum sekundären Amin reduziert.

*Tubulysin-Derivat* ***23***

Ein Tubulysin-Derivat 23 kamm man dadurch gewinnen, daß man Tubulysin 1 in Gegenwart von EDC in Methylenchlorid mit 1-(2-Aminoethyl)-pyrrol-2,5-dion amidiert.

*Tubulysin-Derivat* ***24***

Ein Tubulysin-Derivat 24 mit T = $OR^4$, $R^4$ = $SO_3R^6$ und $R^6$ = H kann man dadurch gewinnen, daß man Tubulysin 1 mit Pyridin-$SO_3$ umsetzt. Analog kann man Tubulysin 1 mit Phosphorsäuredimethylester in Gegenwart von Iod und Pyridin in Methylenchlorid umsetzen.

*Acetyl-Tubulysin-A* ***(25a):*** *R = i-$C_4H_9$, $R^1$ = $CH_3$ (Schema 7)*

8.9 mg Tubulysin A (1) wurden in 200 µl abs. THF gelöst und mit 8.2 µl (30.6 µmol) Acetylchlorid und 7.1 µmol) Triethylamin versetzt. Der Reaktionsansatz wurde 15 min bei Raumtemperatur gerührt, anschließend mit 1 ml Wasser versetzt und dreimal mit Ethylacetat extrahiert. Die vereinigten organischen Phasen wurden eingeengt und am Hochvakuum getrocknet. Das Rohprodukt wurde mittels PSC ($CH_2Cl_2$/MeOH 90/10) gereinigt, wobei 5.6 mg (62 %) 25a erhalten wurden.

**DCI MS** (120 eV, $NH_3$) : 886 $[M+H]^+$; **HRMS (DCI):** $C_{45}H_{67}N_5O_{11}S$: $[M+H]^+$ ber.: 886.4636 (gef.: 886.4701); **$^1$H-NMR** ($CD_3OD$, 300 MHz): δ = 2.58 (m, 1 H, Tut2-H), 1.73 (m, 1 H, Tut3a-H), 2.06 (m, 1 H, Tut3b-H), 4.39 (m, 1 H, Tut4-H), 2.98 (bd, 2 H, Tut5-H), 7.29 (d, 1 H, Tut7-H), 7.00 (d, 1 H, Tut8-H), 1.21 (d, 3 H, Tut10-H), 2.27 (s, 3 H, Tut12-H); **$^{13}$C-NMR** ($CD_3OD$, 75 MHz): δ = 181.1 (TutC1), 38.7 (TutC2), 39.4 (TutC3), 51.1 (TutC4), 41.2 (TutC5), 137.2 (TutC6), 131.4 (TutC7), 122.5 (TutC8), 150.8 (TutC9), 18.8 (TutC10), 171.2 (TutC11), 20.9 (TutC12).

*Isobutyryl-Tubulysin-A* ***(25b):*** *R = i-$C_4H_9$, $R^1$ = $CH(CH_3)_2$ (Schema 7)*

15.1 mg (17.8 µmol) Tubulysin A wurden in 400 µl abs. THF gelöst und mit 5.6 µl (53.4 µmol) *i*-Buttersäurechlorid und 12.5 µl (89.0 µmol) Triethylamin versetzt. Der Reaktionsansatz wurde 30 min bei Raumtemperatur gerührt, anschließend mit 2 ml Wasser versetzt und dreimal mit Ethylacetat extrahiert. Die vereinigten organischen Phasen wurden über Natriumsulfat getrocknet und eingeengt. Die Reinigung des Rohproduktes erfolgt mittels PSC ($CH_2Cl_2$/Methanol 90/10) und ergab 5.3 mg (32 %) **NT20.**

**$R_f$** ($CH_2Cl_2$/MeOH 90/10): 0.36; **Drehwert:** $[\alpha]_D^{20}$ = + 11.5 (c 0.35, Methanol); **IR** (KBr): $\tilde{\nu}$ = 3392 $cm^{-1}$ (m),

2964 (m), 2936 (m), 2875 (w), 1755 (s), 1668 (vs), 1544 (w), 1508 (w), 1468 (w), 1420 (w), 1371 (w), 1227 (s), 1167 (w); **UV** (MeOH): $\lambda_{max}$ (lg ε) = 204 nm (4.54), 223 (sh, 4.20); **DCI MS** (120 eV, $NH_3$): $[M+H]^+$; **ESI MS** (1 eV): 913 $[M+H]^+$; **$^1$H-NMR** ($CD_3OD$, 300 MHz): δ = 2.59 (m, 1 H, Tut2-H), 1.73 (m, 1 H, Tut3a-H), 2.07 (m, 1 H, Tut3b-H), 4.39 (m, 1 H, Tut4-H), 2.98 (bd, 2 H, Tut5-H), 6.99 (d, 2 H, Tut7-H), 7.30 (d, 2 H, Tut8-H), 1.22 (d, 3 H, Tut10-H), 2.82 (d, 1 H, Tut12-H), 1.31 (d, 6 H, Tut13,14-H); **$^{13}$C-NMR** ($CD_3OD$, 75 MHz): δ = 181.1 (TutC1), 38.7 (TutC2), 39.4 (TutC3), 51.1 (TutC4), 41.2 (TutC5), 137.2 (TutC6), 131.4 (TutC7), 122.3 (TutC8), 150.8 (TutC9), 18.8 (TutC10), 177.2 (TutC11), 35.3 (TutC12), 19.2 (TutC13,14).

*Tubulysin A-allylether -allylester **(26a):** R = i-$C_4H_9$, $R^1$ = $CH_2CHCH_2$ (Schema 7)*

6.0 mg (7.1 μmol) Tubulysin A **(1),** gelöst in 300 μl Dichlormethan/Diethylether (1/1), wurden mit 6.2 μl (71.2 μmol) Allylbromid und 6.6 mg (28.5 μmol) Silber(I)oxid versetzt und über Nacht bei Raumtemperatur gerührt. Anschließend wurde über Celite filtriert. Der Rückstand wurde mit Dichlormethan gewaschen und die vereinigten organischen Phasen eingeengt. Die Reinigung des Rohproduktes erfolgte über PSC ($CH_2Cl_2$/MeOH 90/10) und liefert 2.9 mg (44 %) **26a.**

**ESI MS** (1 eV): 924 $[M+H]^+$; **$^1$H-NMR** ($CD_3OD$, 400 MHz): δ = 7.17 (d, 2 H, Tut7-H), 6.86 (d, 2 H, Tut8-H), 1.22 (d, 3 H, Tut10-H), 4.5-4.6 (m, 4 H, Tut11-H Tut14-H), 5.93 (m, 1 H, Tut12-H), 5.1-5.5 (m, 4 H, Tut13-H, Tut16-H), 6.07 (m, 1 H, Tut15-H).

*Tubulysin-A-methylether-methylester **(26b):** R = i-$C_4H_9$, $R^1$ = $CH_3$ (Schema 7)*

21.7 mg (25.7 μmol) Tubulysin A **(1)** wurden in 200 μl Methanol gelöst und bei Raumtemperatur in Abständen von 15 min dreimal mit etherischer Diazomethanlösung versetzt und über Nacht gerührt. Anschließend wurde der Reaktionsansatz zur Trockene gebracht. Die Reinigung erfolgte über PSC ($CH_2Cl_2$/MeOH 90/10) und lieferte 10.3 mg (46 %) **26** und 5.0 mg (23 %) Tubulysin-A-methylester **(20a).**

**DCI MS** (120 eV, $NH_3$): 872 $[M+H]^+$; **HRMS (DCI):** $C_{45}H_{69}N_5O_{10}S$: $[M+H]^+$ ber.: 872.4843 (gef.: 872.4818); **$^1$H-NMR** ($CD_3OD$, 400 MHz): δ = 2.6 (m, 1 H, Tut2-H), 2.86 (d, 2 H, Tut5-H), 7.17 (d, 2 H, Tut7-H), 6.85 (d, 2 H, Tut8-H), 1.19 (d, 3 H, Tut10-H), 3.65 (s, 3 H, Tut11-H), 3.78 (s, 3 H, Tut12-H)

*Tubulysin-A-methylether **(27a):** R = i-$C_4H_9$, $R^1$ = $CH_3$ (Schema 7)*

1.6 mg (1.8 μmol) **26a** ($R^1$ = $CH_3$) wurden in 50 μl DMSO gelöst und mit 700 μl Phosphatpuffer (20 mM $KH_2PO_4$, pH = 7.3) versetzt. Der Reaktionsansatz wurde 5 min ins Ultraschallbad gestellt, anschließend wurden 72 μl Schweineleber-Esterase (Böhringer-Mannheim) zugegeben und 4 h bei 36°C gerührt. Zur Isolierung des Produktes wurde dreimal mit Ethylacetat extrahiert und die vereinigten organischen Phasen wurden zur Trockene gebracht. Eine Reinigung des Rohproduktes erfolgte über PSC ($CH_2Cl_2$/MeOH 90/10) und ergab 0.5 mg (32 %) **27a.**

**DCI MS** (120 eV, $NH_3$): 858 $[M+H]^+$; **HRMS (DCI):** $C_{44}H_{67}N_5O_{10}S$: $[M+H]^+$ ber.: 857.4687 (gef.: 858.4740); **$^1$H-NMR** ($CD_3OD$, 300 MHz): δ = 2.8 (m, 2 H, Tut5-H), 7.19 (d, 2 H, Tut7-H), 6.84 (d, 2 H, Tut8-H), 3.78 (s, 3 H, Tut11-H).

*Iodierung von Tubulysin A **(28a, 29a):** R = i-$C_4H_9$, Hal = I (Schema 8)*

11.0 mg (13.1 μmol) Tubulysin A **(1)** wurden in 200 μl Methanol gelöst und mit 13.0 μl Iodmonochlorid-Lösung (13.1 μmol) versetzt. Der Reaktionsansatz wurde 15 Minuten bei Raumtemperatur gerührt und anschließend direkt über PSC ($CH_2Cl_2$/MeOH 90/10) gereinigt. Dabei wurden 3.1 mg (25 %) **28a** und 3.9 mg (27 %) **29a** erhalten.

**28a:**

**ESI MS** (1 eV): 970 $[M+H]^+$; **$^1$H-NMR** ($CD_3OD$, 300 MHz): δ = 2.58 (m, 1 H, Tut2-H), 1.71 (m, 1-H, Tut3a-H), 2.05 (m, 1-H, Tut3b-H), 4.28 (m, 1-H, Tut4-H), 2.82 (m, 2 H, Tut5-H), 7.10 (dd, 2 H, Tut7-H), 6.75 (d, 2 H, Tut8-H), 7.55 (d, 1 H, Tut12-H); **$^{13}$C-NMR** ($CD_3OD$, 75 MHz): δ = 181.2 (TutC1), 38.7 (TutC2), 39.5 (TutC3), 51.4 (TutC4), 40.6 (TutC5), 132.6 (TutC6), 131.5 (TutC7), 115.6 (TutC8), 156.5 (TutC9), 18.8 (TutC10), 84.4 (TutC11), 141.2 (TutC12).

**29a:**

**ESI MS** (1 eV): 1096 $[M+H]^+$; **$^1$H-NMR** ($CD_3OD$, 600 MHz): δ = 2.58 (m, 1 H, Tut2-H), 1.73 (m, 1 H, Tut3a-H), 2.05 (m, 1 H, Tut3b-H), 4.25 (m, 1 H, Tut4-H), 2.75 (dd, 1 H, Tut5a-H), 2.86 (dd, 1 H, Tut5b-H), 7.61 (s, 2 H, Tut7, 12-H); **$^{13}$C-NMR** ($CD_3OD$, 75 MHz): δ = 181.1 (TutC1), 38.6 (TutC2), 39.6 (TutC3), 51.5 (TutC4), 40.1 (TutC5), 135.8

(TutC6), 141.5 (TutC7,12), 85.1 (TutC8,11), 155.2 (TutC9), 18.8 (TutC10).

*Nitro-Tubulysin A* ***(30a):*** *R = i-$C_4H_9$ (Schema 8)*

Eine Lösung von 12.5 mg (14.8 μmol) Tubulysin A **(1)** in 400 μl Ethanol wurde mit 100 μl Eisessig und 20.5 mg (296.6 μmol) Natriumnitrit, gelöst in 100 μl Wasser, versetzt. Der Reaktionsansatz wurde zwei Tage bei Raumtemperatur gerührt und anschließend am Hochvakuum zur Trockene gebracht. Das Rohprodukt wurde mittels PSC ($CH_2Cl_2$/MeOH 90/10) gereinigt, wobei 9.8 mg (74 %) **30** erhalten wurden.

**IR** (KBr): $\tilde{\nu}$ = 3411 $cm^{-1}$ (m), 2962 (m), 2932 (m), 2873 (w), 1741 (s), 1666 (vs), 1539 (s), 1492 (w), 1424 (w), 1370 (w), 1223 (s); **UV** (MeOH): $\lambda_{max}$ (lg ε) = 205 nm (4.56), 216 (sh, 4.42), 234 (sh, 4.19), 274 (3.77), 360 (3.43); **DCI MS** (120 eV, $NH_3$): 889 $[M+H]^+$; **$^1$H-NMR** ($CD_3OD$, 400 MHz): δ = 2.60 (m, 1 H, Tut2-H), 1.74 (m, 1 H, Tut3a-H), 2.09 (m, 1 H, Tut3b-H), 4.37 (ddd, 2 H, Tut4-H), 2.91 (dd, 1 H, Tut5a-H), 3.01 (dd, 1 H, Tut5b-H), 7.56 (dd, 1 H, Tut7-H), 7.07 (d, 1 H, Tut8-H), 1.27 (d, 3 H, Tut10-H), 7.97 (d, 1 H, Tut12-H); **$^{13}$C-NMR** ($CD_3OD$, 400 MHz): δ = 180.7 (TutC1), 38.5 (TutC2), 39.6 (TutC3), 51.0 (TutC4), 40.7 (TutC5), 132.0 (TutC6), 139.4 (TutC7), 120.8 (TutC8), 154.2 (TutC9), 18.8 (TutC10), 135.3 (TutC11), 126.5 (TutC12).

*Tubulysin-Derivate* ***31 und 32***

Ein Tubulysin-Derivat 31 kann man dadurch gewinnen, daß man das Nitro-Tubulysin A (30a) in Ethanol mit elementarem Wasserstoff mit einem Pd/C-Katalysator katalytisch reduziert.

Das gewonnen Tubulysin-Deriat 31 kann man mit Essigsäureanhydrid zu einem Tubulysin-Derivat 32 acylieren.

*Tubulysin A-N-oxid* ***(33a):*** *R = i-$C_4H_9$ (Schema 9)*

9.9 mg (11.7 μmol) Tubulysin A **(1)** wurden in 200 μl Dichlormethan gelöst, mit 290 μl (11.7μmol) *m*-CPBA-Lösung (10 mg/ml Dichlormethan) versetzt und 30 Minuten bei Raumtemperatur gerührt. Nachdem der Reaktionsansatz reduziert wurde, erfolgte direkt die Reinigung mittels PSC ($CH_2Cl_2$/Methanol 85/15), wobei 5.2 mg (52 %) **33a** erhalten wurden.

**ESI MS** (1 eV): 860 $[M+H]^+$.

*Tubulysin-Derivat* ***34***

Ein Tubulysin-Derivat 34 kann man dadurch gewinnen, daß man Tubulysin A-N-oxid (33a) bei etwa 75 ˚C mit Essigsüreanhydrid behandelt.

Abkürzungen

| Abkürzung | Name |
|---|---|
| $C_5Cl_5NF$-Triflat | *N*-Fluorpentachlorpyridinium-Triflat |
| $CH_3CN$ | Acetonitril |
| DAST | Diethylaminoschwefeltrifluorid |
| DMAP | Dimethylaminopyridin |
| EDC | *N*-Ethyl-*N'*-(3-dimethylaminopropyl)-carbodiimid |
| ICl | Iodmonochlorid |
| *m*-CPBA | meta-Chlorperbenzoesäure |
| $Me_3SiCl$ | Trimethylchlorsilan |
| $NaCNBH_3$ | Natriumcyanoborhydrid |
| NBS | *N*-Bromsuccinimid |
| NMO | *N*-Methyl-morpholin-*N*-oxid |
| *p*-$CH_3$-$C_6H_4SO_2OH$ | *para*-Toluolsulfonsäure |
| TFA | Trifluoressigsäure |
| THF | Tetrahydrofuran |
| TPAP | *tetra*-Propylammoniumperruthenat |

## Patentansprüche

1. Verbindung der folgenden allgemeinen Formel I (Tubulysin):

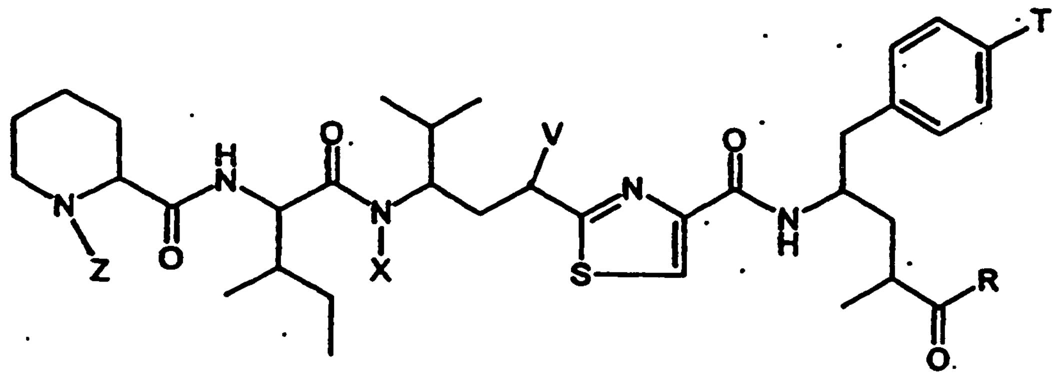

mit den folgenden Bedeutungen für R, $R^1$, $R^4$, $R^5$, $R^6$ $R^7$, $R^8$, $R^9$, $R^{10}$, $R^{11}$, T, V, X und Z:

R = $OR^1$
$R^1$ = Alkyl oder Aryl
T = H oder $OR^4$
$R^4$ = H, Alkyl, Aryl, $COR^5$, P(O) $(OR^6)_2$ oder $SO_3R^6$
$R^5$ = Alkyl, Alkenyl, Aryl oder Heteroaryl
$R^6$ = H, Alkyl oder Metallion
V = $OR^7$
$R^7$ = $COR^8$
$R^8$ = Alkyl, Alkenyl oder Aryl
X = $CH_2OR^9$
$R^9$ = $COR^{10}$
$R^{10}$ = Alkyl, Alkenyl, Aryl oder Heteroaryl
$R^{11}$ = Alkyl, $CF_3$ oder Aryl und/oder
Z = $CH_3$ oder $COR^{11}$.

2. Verbindung nach Anspruch 1 mit
R, $R^1$, $R^4$, $R^5$, $R^8$, $R^{10}$ und/oder $R^{11}$ = unsubstituiertes oder substituiertes Phenyl, insbesondere $C_{1-4}$-Alkyl-substituiertes Phenyl
$R^5$ = $C_{1-4}$-Alkyl, $C_{2-6}$-Alkenyl oder Pyridyl
$R^5$ und/oder X = $C_{2-4}$-Alkenyl
$R^6$ = Alkalimetall-Ion, insbesondere Na-Ion, oder Erdalkalimetall-Ion
$R^8$ = $C_{2-4}$-Alkenyl und/oder
$R^{10}$ = $C_{2-6}$-Alkenyl, insbesondere $C_{2-4}$-Alkenyl, oder Pyridyl.

3. Therapeutisches Mittel, insbesondere Cytostatikum, mit ein oder mehreren Verbindungen gemäß Anspruch 1 oder 2 als Wirkstoff neben einem oder mehreren fakultativen üblichen Trägern und/oder einem oder mehreren fakultativen üblichen Verdünnungsmitteln.

4. Verbindung nach Anspruch 1 oder 2, wobei
Alkyl verzweigtes, unverzweigtes oder cyclisches $C_{1-20}$-Alkyl, insbesondere $C_{1-7}$-Alkyl, vorzugsweise $C_{1-6}$-Alkyl und besonders bevorzugt $C_{1-4}$-Alkyl ist, insbesondere Methyl, Ethyl, Propyl, Isopropyl, n-Butyl, iso-Butyl, sek-Butyl, tert-Butyl, und wobei Cycloalkyl vorzugsweise 3 bis 8 C-Atome im Ring besitzt.

5. Verbindung nach Anspruch 1, 2 oder 4, wobei

Alkenyl verzweigtes, unverzweigtes oder cyclisches $C_{2-20}$-Alkenyl, insbesondere $C_{2-7}$-Alkenyl, vorzugsweise $C_{2-6}$-Alkenyl und besonders bevorzugt $C_{2-4}$-Alkenyl ist, insbesondere Vinyl, Allyl, Propen-1-yl, Propen-2-yl, But-1-en-1-yl, But-1-en-2-yl, But-1-en-3-yl, But-1-en-4-yl, But-2-en-1-yl, But-2-en-2-yl, 2-Methyl-propen-1-yl, 2-Methyl-propen-3-yl ist, und wobei Cycloalkenyl vorzugsweise 3 bis 8 C-Atome im Ring besitzt, und die Anzahl der Doppelbindungen der Alkenylgruppen 1 bis 3 beträgt.

6. Verbindung nach Anspruch 1, 2, 4 oder 5, wobei
Aryl Phenyl, Naphthyl oder Biphenylyl bedeutet.

7. Verbindung nach Anspruch 1, 2, 4, 5 oder 6, wobei
Heteroaryl Furyl, Thienyl, Imidazolyl, Indolyl, Pyridyl, Pyridinyl, Pyrrolyl, Thiazolyl, Oxazolyl, oder Pyrimidinyl bedeutet.

8. Verbindung nach Anspruch 1, 2, 4, 5, 6 oder 7, wobei
Alkyl, Alkenyl, Aryl und Heteroaryl unsubstituiert oder substituiert sind und insbesondere in beliebiger Position 1 bis 3 Substituenten aus der durch $C_{1-3}$-Alkyl, $C_{1-3}$-Alkyloxy, Hydroxy, Amino ($NH_2$) oder Nitro ($NO_2$) gebildeten Gruppen tragen.

## Claims

1. Compound of the following general formula I (tubulysin):

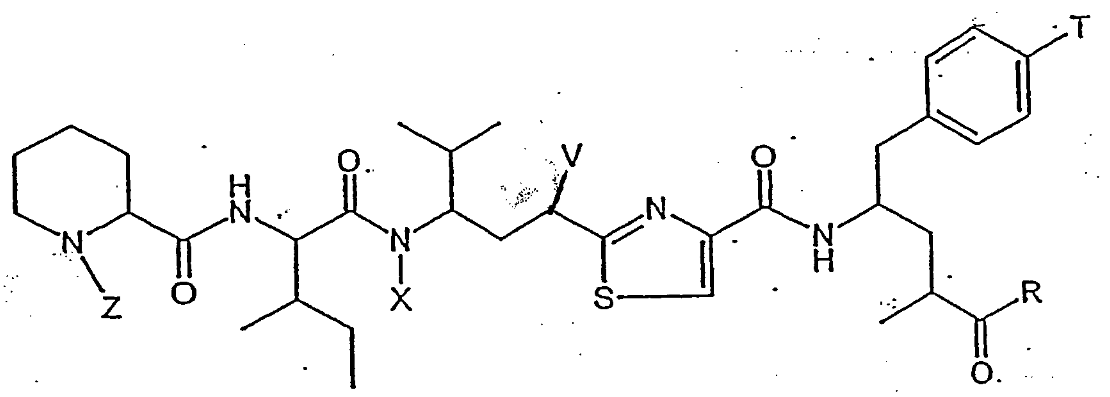

R, $R^1$, $R^4$, $R^5$, $R^6$, $R^7$, $R^8$, $R^9$, $R^{10}$, $R^{11}$, T, V, X and Z having the following meanings:

R = $OR^1$
$R^1$ = alkyl or aryl
T = H or $OR^4$
$R^4$ = H, alkyl, aryl, $COR^5$, $P(O)(OR^6)_2$ or $SO_3R^6$
$R^5$ = alkyl, alkenyl, aryl or heteroaryl
$R^6$ = H, alkyl or a metal ion
V = $OR^7$
$R^7$ = $COR^8$
$R^8$ = alkyl, alkenyl or aryl
X = $CH_2OR^9$
$R^9$ = $COR^{10}$
$R^{10}$ = alkyl, alkenyl, aryl or heteroaryl
$R^{11}$ = alkyl, $CF_3$ or aryl and/or
Z = $CH_3$ or $COR^{11}$.

2. Compound according to claim 1, wherein
R, $R^1$, $R^4$, $R^5$, $R^8$, $R^{10}$ and/or $R^{11}$ = unsubstituted or substituted phenyl, especially $C_{1-4}$alkylsubstituted phenyl
$R^5$ = $C_{1-4}$alkyl, $C_{2-6}$alkenyl or pyridyl

$R^5$ and/or X = $C_{2-4}$alkenyl
$R^6$ = an alkali metal ion, especially the Na ion, or an alkaline earth metal ion
$R^8$ = $C_{2-4}$alkenyl and/or
$R^{10}$ = $C_{2-6}$alkenyl, especially $C_{2-4}$alkenyl, or pyridyl.

**3.** Therapeutic preparation, especially a cytostatic agent, comprising one or more compounds according to claim 1 or 2 as active ingredient in addition to one or more optional customary carriers and/or one or more optional customary diluents.

**4.** Compound according to claim 1 or 2, wherein
alkyl is branched, unbranched or cyclic $C_{1-20}$alkyl, especially $C_{1-7}$alkyl, preferably $C_{1-6}$alkyl and more preferably $C_{1-4}$alkyl, especially methyl, ethyl, propyl, isopropyl, n-butyl, isobutyl, sec-butyl, tert-butyl, and cycloalkyl having preferably from 3 to 8 carbon atoms in the ring.

**5.** Compound according to claim 1, 2 or 4, wherein
alkenyl is branched, unbranched or cyclic $C_{2-20}$alkenyl, especially $C_{2-7}$alkenyl, preferably $C_{2-6}$alkenyl and more preferably $C_{2-4}$alkenyl, especially vinyl, allyl, propen-1-yl, propen-2-yl, but-1-en-1-yl, but-1-en-2-yl, but-1-en-3-yl, but-1-en-4-yl, but-2-en-1-yl, but-2-en-2-yl, 2-methyl-propen-1-yl, 2-methyl-propen-3-yl, and cycloalkenyl having preferably from 3 to 8 carbon atoms in the ring and the number of double bonds in the alkenyl groups being from 1 to 3.

**6.** Compound according to claim 1, 2, 4 or 5, wherein
aryl is phenyl, naphthyl and biphenylyl.

**7.** Compound according to claim 1, 2, 4, 5 or 6, wherein
heteroaryl is furyl, thienyl, imidazolyl, indolyl, pyridyl, pyridinyl, pyrrolyl, thiazolyl, oxazolyl or pyrimidinyl.

**8.** Compound according to claim 1, 2, 4, 5, 6 or 7, wherein
alkyl, alkenyl, aryl and heteroaryl are unsubstituted or substituted and, especially, carry, in any position, from 1 to 3 substituents from the group formed by $C_{1-3}$alkyl, $C_{1-3}$alkoxy, hydroxy, amino ($NH_2$) or nitro ($NO_2$).

## Revendications

**1.** Composé de formule générale I suivante (tubulysine) :

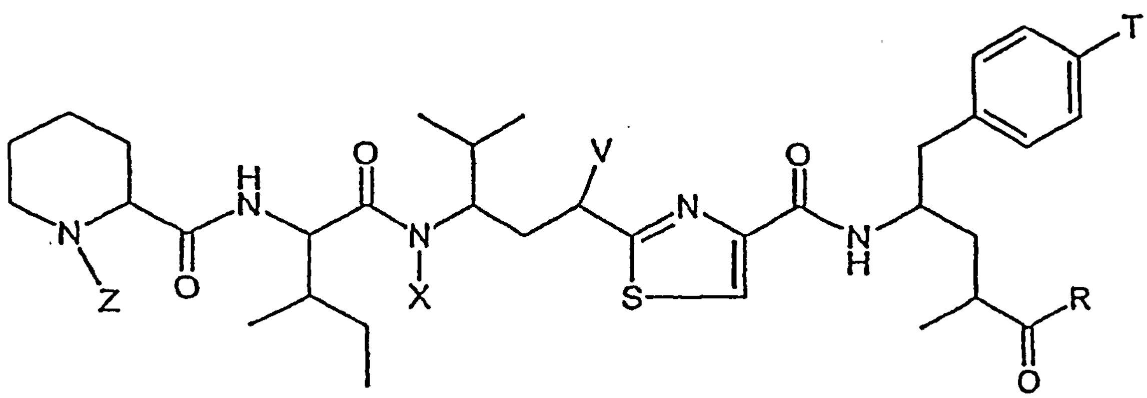

avec les significations suivantes pour R, $R^1$, $R^4$, $R^5$, $R^6$, $R^7$, $R^8$, $R^9$, $R^{10}$, $R^{11}$, T, V, X et Z :

R = $OR^1$
$R^1$ = alkyle ou aryle
T = H ou $OR^4$
$R^4$ = H, alkyle, aryle, $COR^5$, $P(O)(OR^6)_2$ ou $SO_3R^6$
$R^5$ = alkyle, alcényle, aryle ou hétéroaryle
$R^6$ = H, alkyle ou un ion métallique

$V = OR^7$
$R^7 = COR^8$
$R^8$ = alkyle, alcényle ou aryle
$X = CH_2OR^9$
$R^9 = COR^{10}$
$R^{10}$ = alkyle, alcényle, aryle ou hétéroaryle
$R^{11}$ = alkyle, $CF_3$ ou aryle et/ou
$Z = CH_3$ ou $COR^{11}$.

**2.** Composé selon la revendication 1 avec
R, $R^1$, $R^4$, $R^5$, $R^8$, $R^{10}$ et/ou $R^{11}$ = phényle substitué ou non substitué, notamment phényle substitué par alkyle en $C_{1-4}$
$R^5$ = alkyle en $C_{1-4}$, alcényle en $C_{2-6}$ ou pyridyle
$R^5$ et/ou X = alcényle en $C_{2-4}$
$R^6$ = un ion métallique, notamment un ion Na, ou un ion de métal alcalino-terreux
$R^8$ = alcényle en $C_{2-4}$ et/ou
$R^{10}$ = alcényle en $C_{2-6}$, notamment alcényle en $C_{2-4}$ ou pyridyle.

**3.** Agent thérapeutique, en particulier cytostatique, avec un ou plusieurs composés selon la revendication 1 ou 2 à titre de principe actif, en plus d'un ou de plusieurs véhicules usuels facultatifs et/ou d'un ou de plusieurs agents diluants usuels facultatifs.

**4.** Composé selon la revendication 1 ou 2, dans lequel alkyle est alkyle en $C_{1-20}$ ramifié, non ramifié ou cyclique, en particulier alkyle en $C_{1-7}$, de préférence alkyle en $C_{1-6}$ et particulièrement préférentiellement alkyle en $C_{1-4}$, en particulier méthyle, éthyle, propyle, isopropyle, n-butyle, isobutyle, sec.-butyle, tert.-butyle, et dans lequel cycloalkyle possède de préférence 3 à 8 atomes de C dans le cycle.

**5.** Composé selon la revendication 1, 2 ou 4, dans lequel alcényle est alcényle en $C_{2-20}$ ramifié, non ramifié ou cyclique, en particulier alcényle en $C_{2-7}$, de préférence alcényle en $C_{2-6}$ et particulièrement préférentiellement alcényle en $C_{2-4}$, en particulier vinyle, allyle, propèn-1-yle, propèn-2-yle, but-1-èn-1-yle, but-1-èn-2-yle, but-1-èn-3-yle, but-1-èn-4-yle, but-2-èn-1-yle, but-2-èn-2-yle, 2-méthyl-propèn-1-yle, 2-méthyl-propèn-3-yle, et dans lequel cycloalcényle possède de préférence 3 à 8 atomes de C dans le cycle, et le nombre de doubles liaisons des groupes alcényles va de 1 à 3.

**6.** Composé selon la revendication 1, 2, 4 ou 5, dans lequel aryle signifie phényle, naphtyle ou biphénylyle.

**7.** Composé selon la revendication 1, 2, 4, 5 ou 6, dans lequel hétéroaryle signifie furyle, thiényle, imidazolyle, indolyle, pyridyle, pyridinyle, pyrrolyle, thiazolyle, oxazolyle ou pyrimidinyle.

**8.** Composé selon la revendication 1, 2, 4, 5, 6 ou 7, dans lequel alkyle, alcényle, aryle et hétéroaryle sont substitués ou non substitués et portent 1 à 3 substituants issus des groupes formés par alkyle en $C_{1-3}$, alkyloxy en $C_{1-3}$, hydroxy, amino ($NH_2$) ou nitro ($NO_2$).

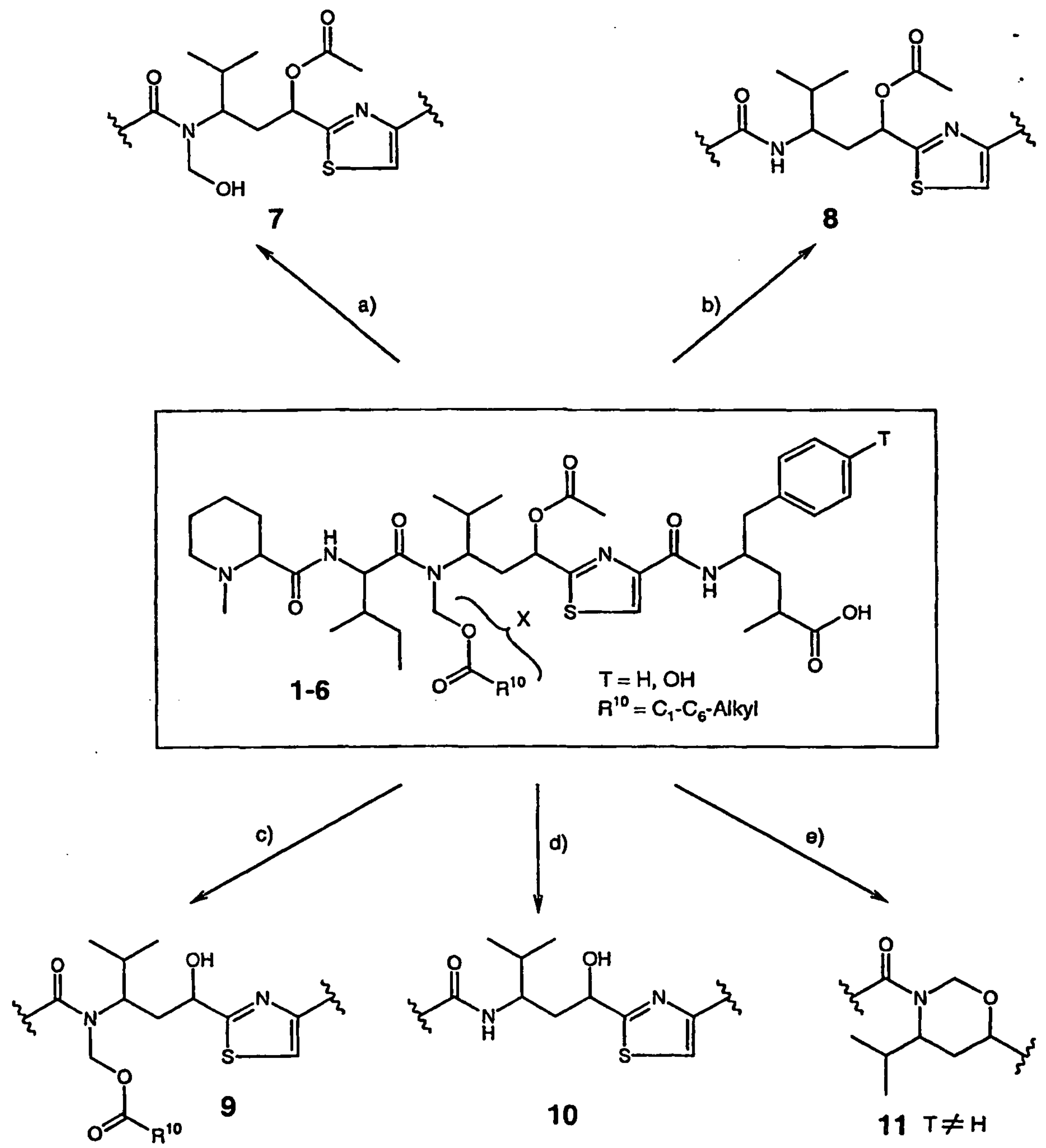


7

8

1-6

9

10

11 T≠H

a) 0.1 M HCl, Dioxan, 50°C; b) 0.1 M HCl, 100°C; c) $NH_3$, MeOH; d) 1 M NaOH, MeOH; e) 0.5 M HCl, 100°C

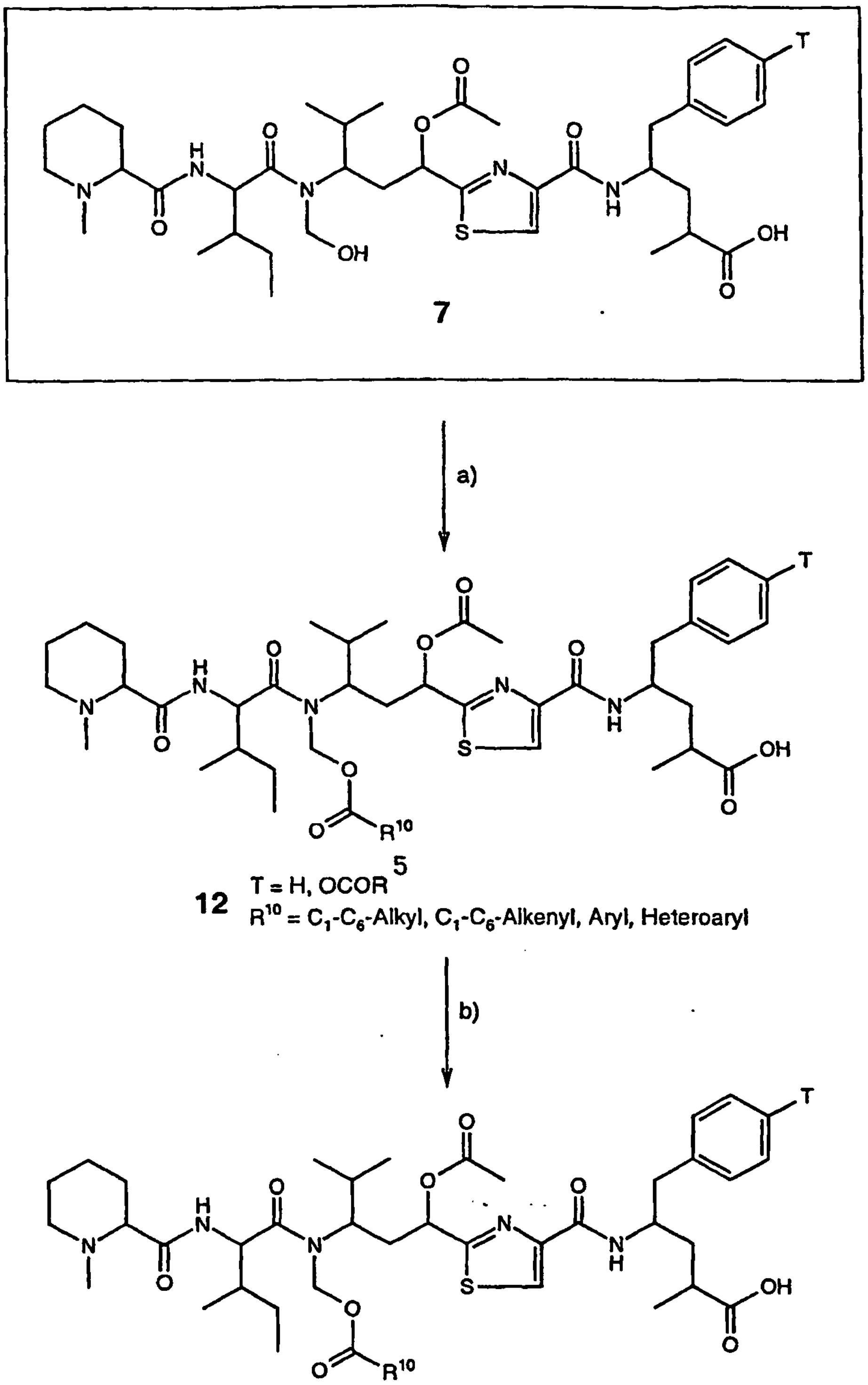


**7**

**12** T = H, $OCOR^5$
$R^{10} = C_1$-$C_6$-Alkyl, $C_1$-$C_6$-Alkenyl, Aryl, Heteroaryl

**13** T = H, OH
$R^{10} = C_1$-$C_6$-Alkyl, $C_1$-$C_6$-Alkenyl, Aryl, Heteroaryl

a) $R^{10}COCl$, $Et_3N$; b) $NH_3$

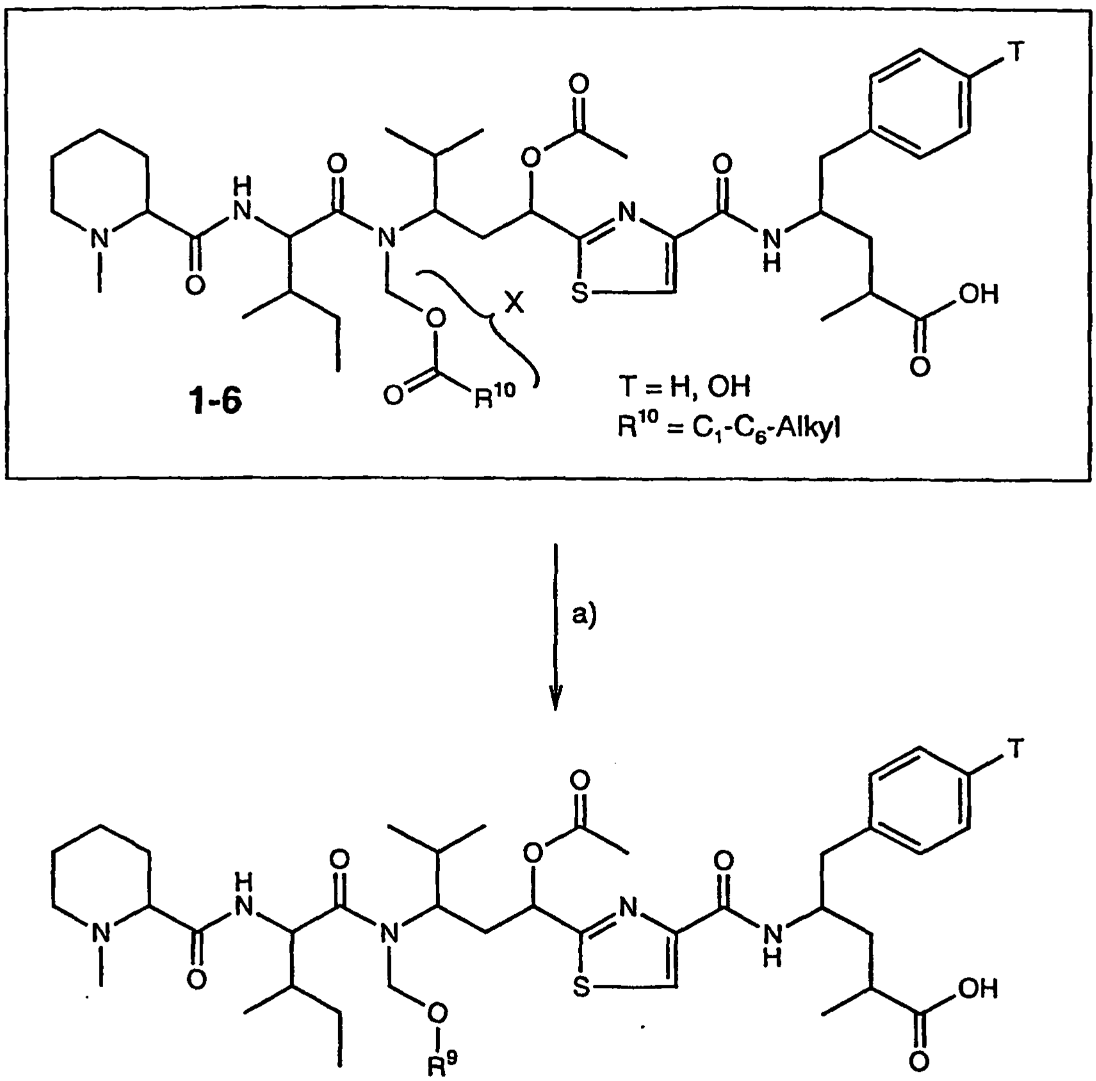


**14** $R^9 = C_1$-$C_4$-Alkyl, Alkenyl, Aryl

a) *p*-$CH_3$-$C_6H_4SO_2OH$, $R^9OH$, THF, 80°C

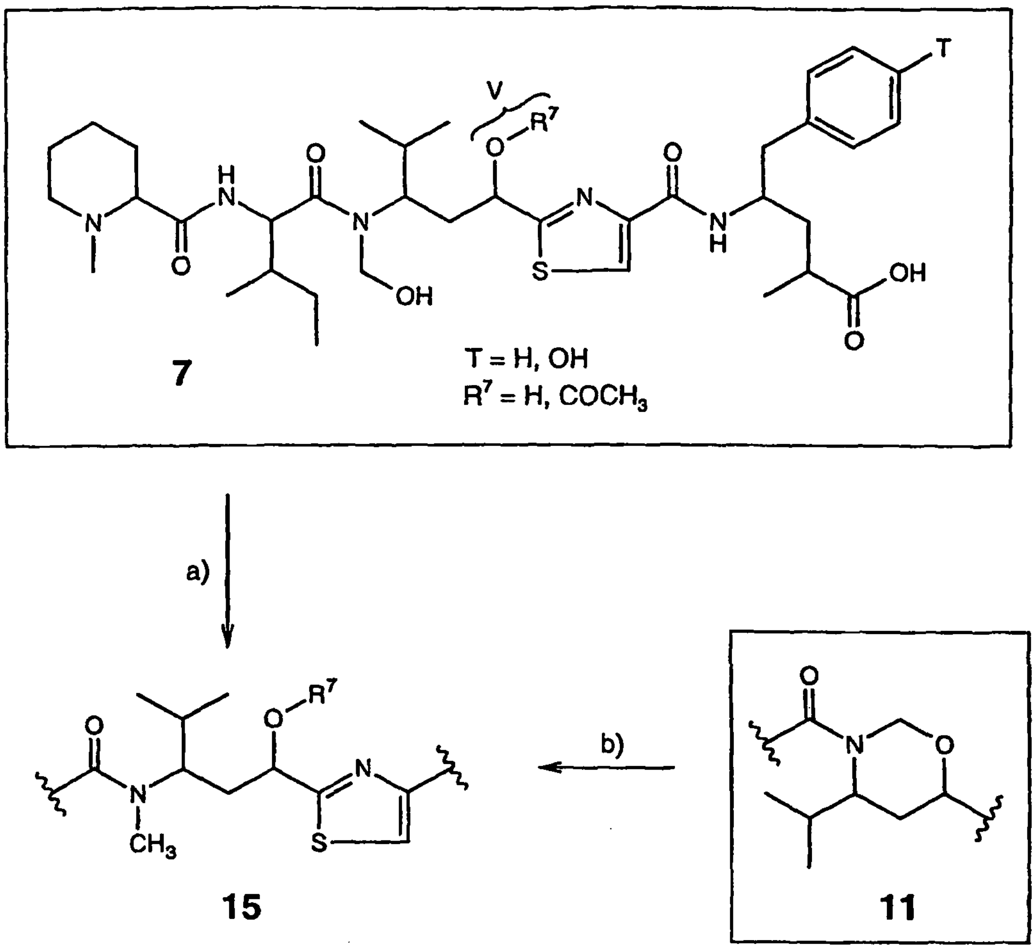


7

T = H, OH

$R^7$ = H, $COCH_3$

15

11

a) $NaCNBH_3$, TFA, MeOH; b) $NaCNBH_3$, $Me_3SiCl$, $CH_3CN$

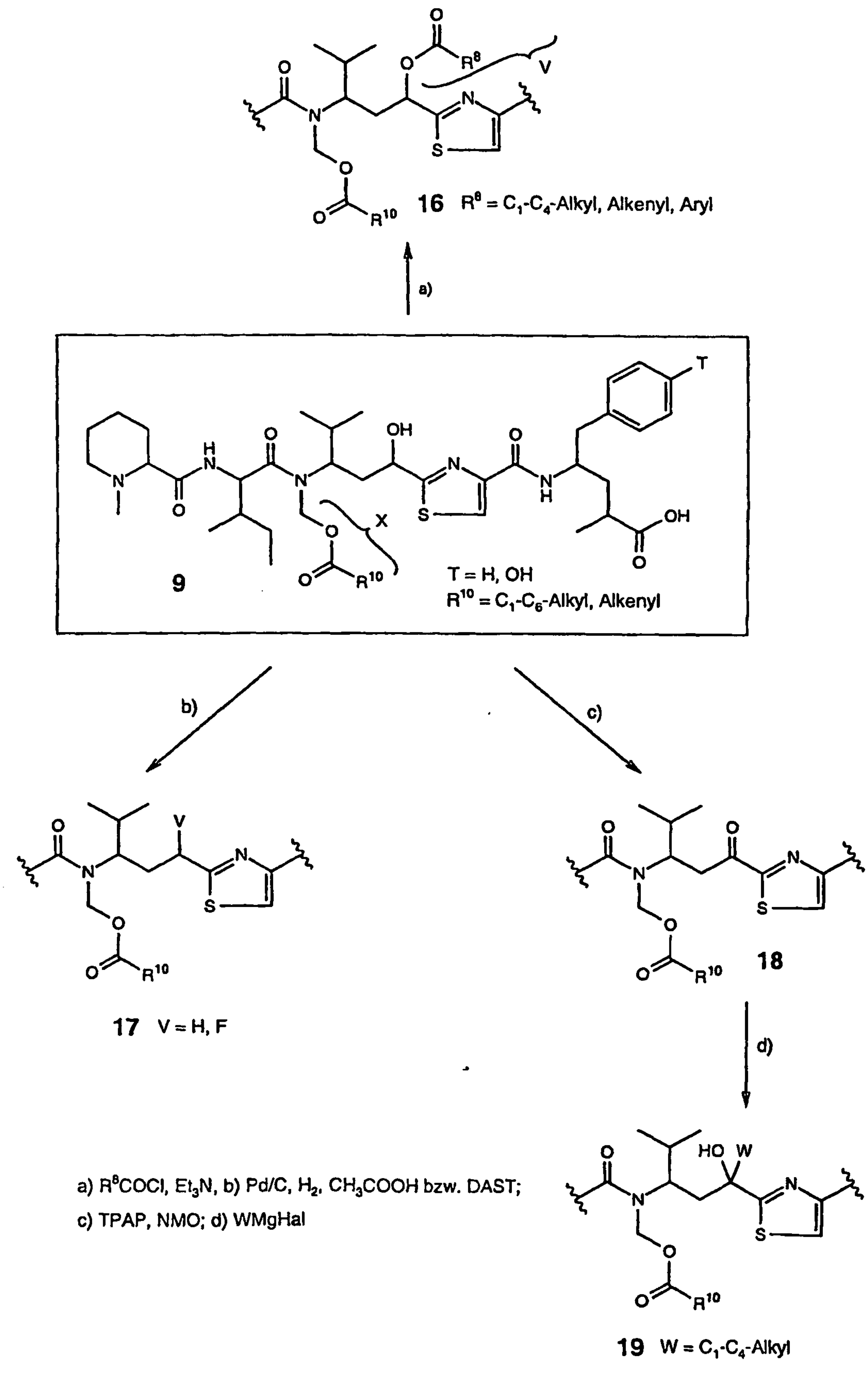


**16** $R^8 = C_1$-$C_4$-Alkyl, Alkenyl, Aryl

**9** T = H, OH

$R^{10} = C_1$-$C_6$-Alkyl, Alkenyl

**17** V = H, F

**18**

**19** W = $C_1$-$C_4$-Alkyl

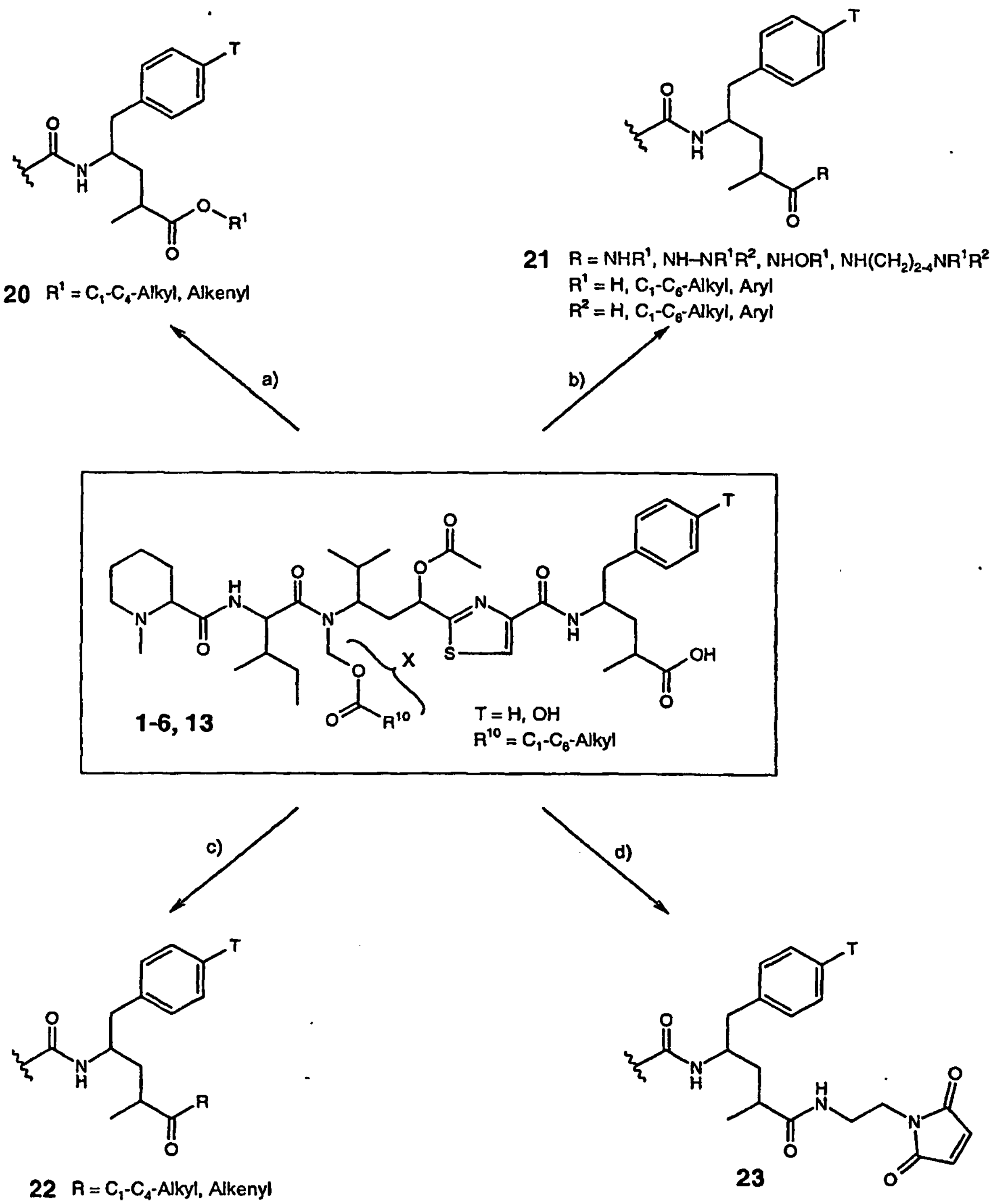


**20** $R^1 = C_1$-$C_4$-Alkyl, Alkenyl

**21** $R = NHR^1$, $NH$–$NR^1R^2$, $NHOR^1$, $NH(CH_2)_{2\text{-}4}NR^1R^2$
$R^1 = H$, $C_1$-$C_6$-Alkyl, Aryl
$R^2 = H$, $C_1$-$C_6$-Alkyl, Aryl

**1-6, 13**

T = H, OH
$R^{10} = C_1$-$C_6$-Alkyl

**22** $R = C_1$-$C_4$-Alkyl, Alkenyl

**23**

a) EDC, $R^1OH$, DMAP, $CH_2Cl_2$; b) EDC, RH, $CH_2Cl_2$ oder *i*-Butylchlorformiat, $Et_3N$, RH, abs. THF
c) RLi; d) EDC, 1-(2-Aminoethyl)-pyrrol-2,5-dion,$CH_2Cl_2$

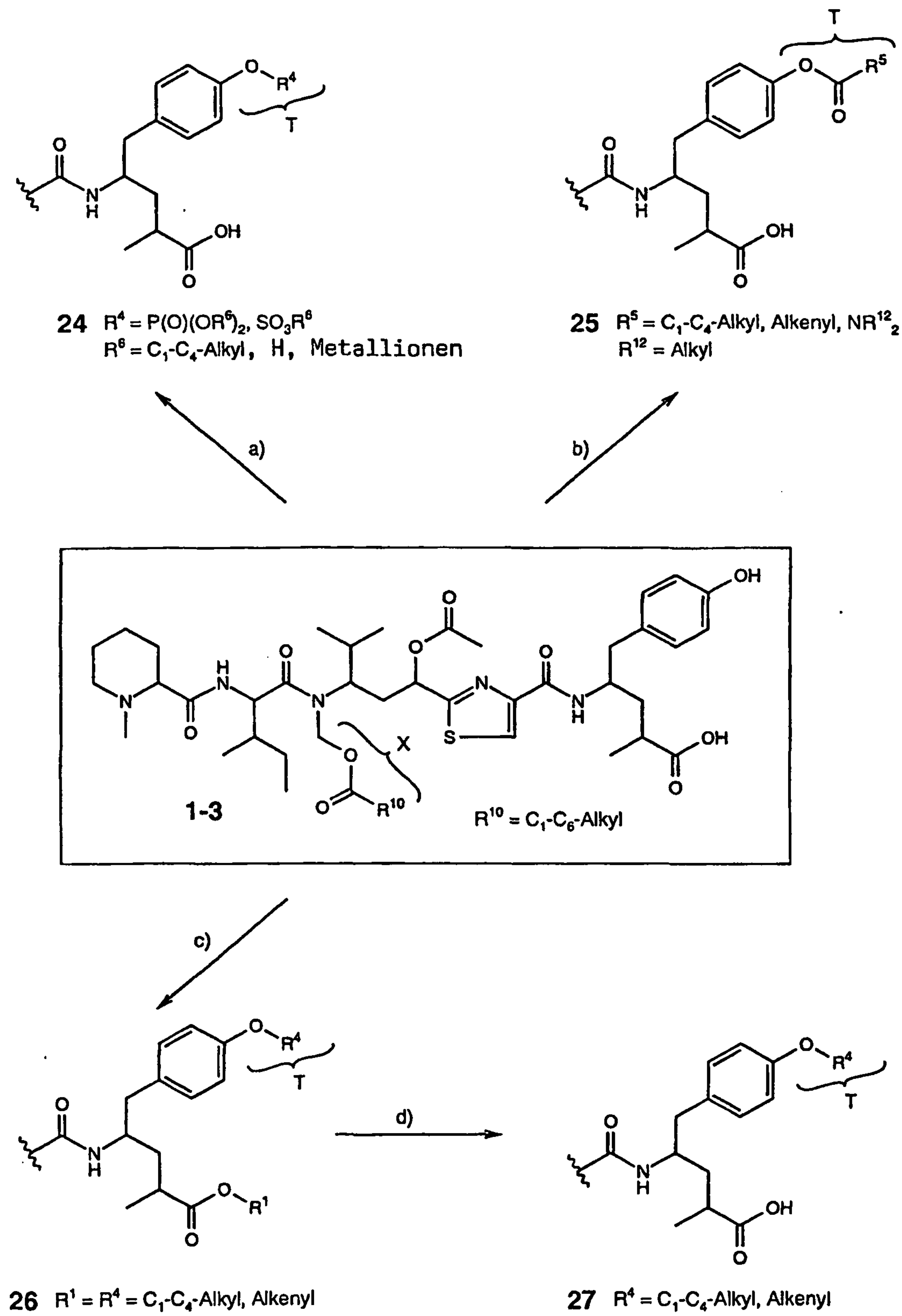


a) $P(O)(OR^6)_2OH$, $I_2$, Pyridin, $CH_2Cl_2$ bzw. Pyridin-$SO_3$; b) $R^5COCl$, $Et_3N$, abs. THF;

c) $Ag_2O$, $R^4I$, $CH_2Cl_2$; für $R^4 = CH_3$: $CH_2N_2$, MeOH; d) Schweineleber-Esterase, $KH_2PO_4$-Puffer, 36°C;

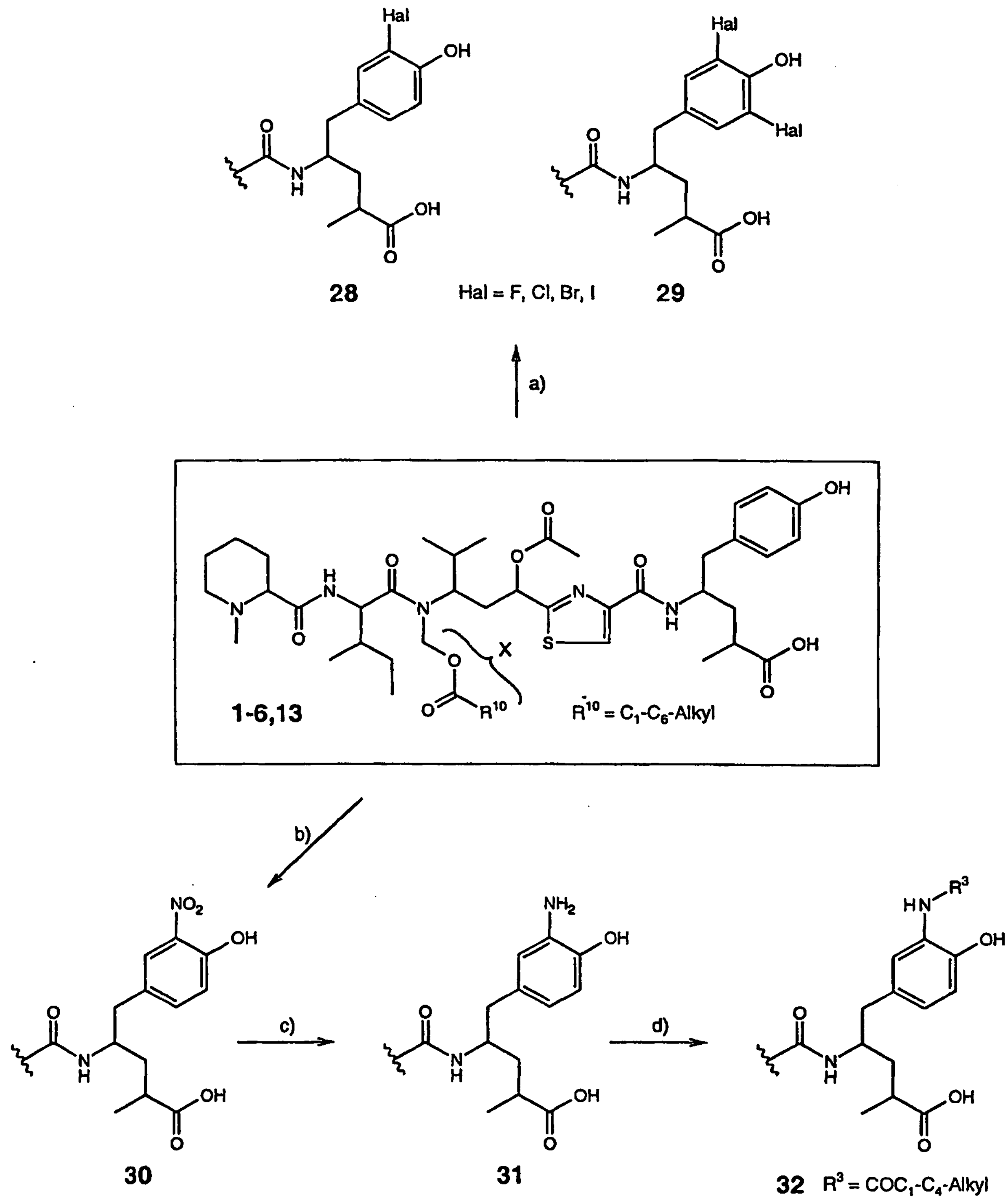


28, 29, 1-6,13, 30, 31, 32

a) $C_5Cl_5NF$-triflat, $SO_2Cl_2$, NBS, ICl; b) $NaNO_2$, $CH_3COOH$, EtOH; c) Pd/C, $H_2$, EtOH; d) $(R^3CO)_2O$

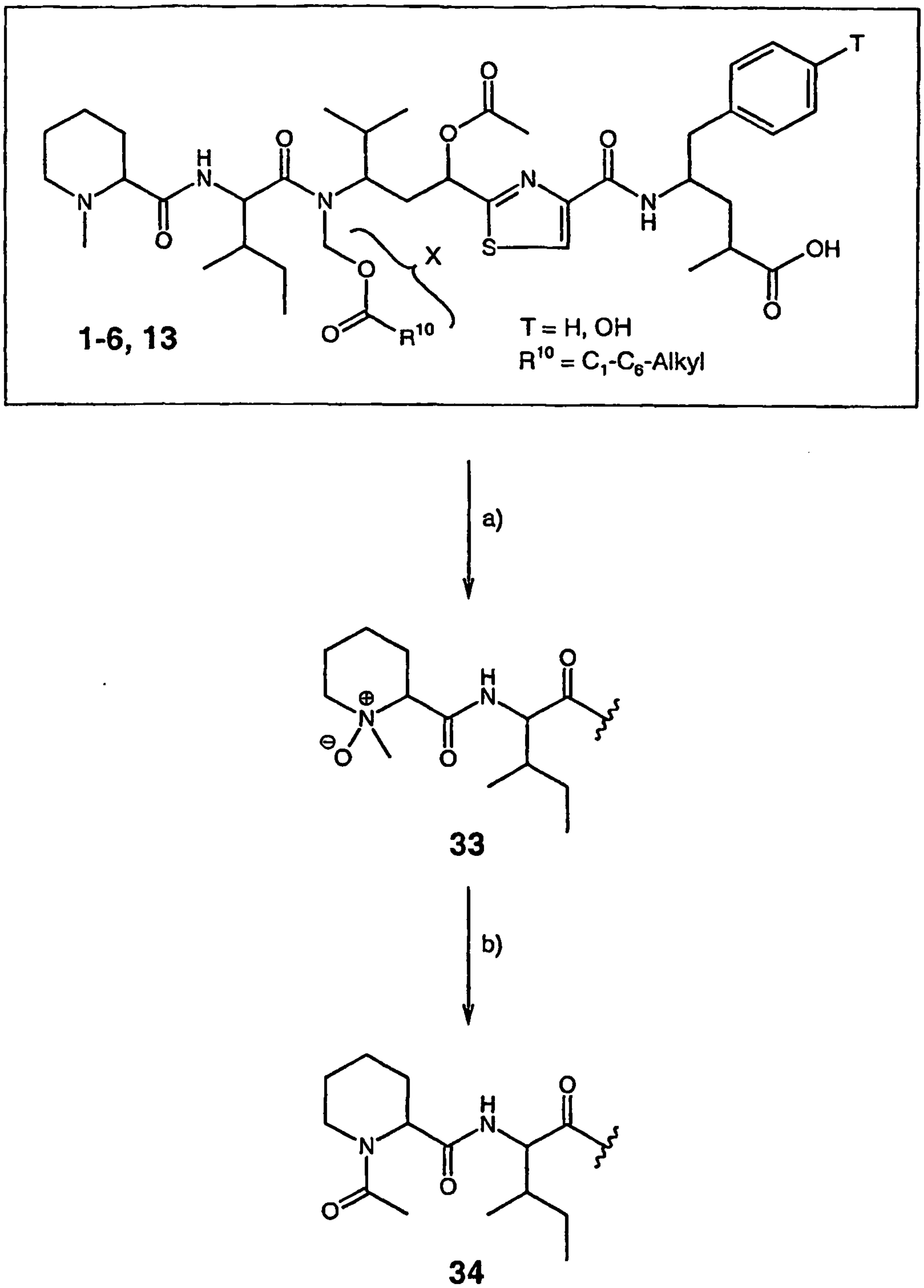


**1-6, 13**

**33**

**34**

a) *m*-CPBA, $CH_2Cl_2$; b) $Ac_2O$, 75°C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19638870 A1, H. Reichenbach, G. Höfle, F. Sasse, H. Steinmetz (GBF)
- DE 1996 A1


### In der Beschreibung aufgeführte Nicht-Patentliteratur


- **F. SASSE ; H. STEINMETZ ; J. HEIL ; G. HÖFLE ; H. REICHENBACH.** *J. Antibiot.,* 2000, vol. 53, 579-558